# FASCICULE DE BREVET EUROPEEN

(11) **EP 0 626 014 B1**
(45) Date de publication et mention de la délivrance du brevet: **24.10.2001**
(21) Numéro de dépôt: 93905378.1
(22) Date de dépôt: 09.02.1993
(51) Int. Cl.: C12N 15/82, C12N 15/54, A01H 1/00, A01H 5/00

(54) **PLANTE PORTANT DES GENES CODANT POUR DES ENZYMES DE LA VOIE DE BIOSYNTHESE DES PHYTOSTEROLS, ET PROCEDE D'OBTENTION**
FÜR DIE ENZYME DES PHYTOSTEROLSBIOSYNTHESEWEGS KODIERENDE GENE ENHALTENDE PFLANZE UND VERFAHREN ZU IHRER HERSTELLUNG
PLANT CARRYING GENES CODING FOR ENZYMES OF THE PHYTOSTEROL BIOSYNTHESIS PATHWAY AND PROCESS FOR THE PRODUCTION OF SAME

(30) Priorité: 14.02.1992 FR 9201712
(43) Date de publication de la demande: 30.11.1994
(73) Titulaire: VERNEUIL RECHERCHE, 77390 Verneuil-l'Etang (FR)
(72) Inventeur: LEJEUNE, Fabienne, F-86000 Poitiers (FR); TOURTE, Monique, F-86190 Beruges (FR); OULMOUDEN, Ahmad, F-86000 Poitiers (FR); KARST, Francis, F-86000 Poitiers (FR)
(74) Mandataire: Michelet, Alain
(86) Numéro de dépôt international: FR9300134
(87) Numéro de publication internationale: WO9316187

(56) Documents cités:
- EP-A- 0 409 628
- EP-A- 0 480 730
- WO-A-91/13078
- PLANT PHYSIOLOGY.ANNUAL MEETING OF THE AMERICAN SOCIETY OF PLANT PHYSIOLOGISTS, ALBUQUERQUE, NEW MEXICO, JULY 28 - AUGUST 1, 1991. vol. 96, no. 1, Mai 1991, ROCKVILLE, MD, USA. page 127 CHAPPELL, J., ET AL. 'Is HMG-Co A reductase a rate limiting step for isoprenoid metabolism?'

## Description

La présente invention a pour objet des plantes portant des gènes codant pour une ou plusieurs des enzymes des étapes précoces de la voie de biosynthèse des phytostérols .

Elle est également relative à un procédé d'obtention desdites plantes .

L'acide mévalonique (AMV) est un précurseur des unités isoprènes en C₅ qui sont elles-mêmes à la base de la biosynthèse d'une part des phytostérols , et d'autre part de nombreuses molécules présentant une activité physiologique fondamentale dans le métabolisme, la prolifération et la différenciation cellulaires des végétaux : régulateurs de croissance, caroténoïdes , chaîne phytol de la chlorophylle, ubiquinone.

La première étape de métabolisation de l'AMV est sa phorphorylation par la mévalonate kinase (MK). L'AMV est ensuite converti en isopentényl pyrophosphate (IPP), molécule dont dérivent les cytokinines (CKs), et qui constitue l'unité isoprénique d'enchaînement menant en particulier à la synthèse des gibbéréllines (GAs) et de l'acide abscissique (ABA). Les voies de biosynthèse simplifiées de ces trois catégories de phytohormones sont schématisées sur les figures lA et 1B . Ces phytohormones sont particulièrement intéressantes du fait de leur implication dans les phénomènes de régénération;

Le point de départ de la voie de biosynthèse des GAs est l'AMV. Une suite de réactions aboutit ensuite à la cyclisation du géranyl-géranyl pyrophosphate (GGPP) et à la formation de kaurène qui est oxydé et réarrangé pour donner l'aldéhyde de GA₁₂ ( ROBERTS et HOOLEY, Plant Growth Regulators 1988 ed: Blakie, Chapman & Hall, New York,) , produit dont dérivent les diverses GAs.

Les sites de synthèse GAs se situent chez les très jeunes feuilles, dans les bourgeons en activité, aux extrémités des racines et au niveau des embryons . Leur circulation se fait par la voie libérienne ; les GAs sont souvent liées à des sucres et sont libérées au niveau des sites d'action.

Les principales propriétés des GAs sont :
- un effet net sur l'élongation des entrenoeuds. Cet effet peut aussi se produire sur les pédoncules floraux, ce qui permet d'obtenir soit une plus grande précocité soit des inflorescences plus développées ,
- un effet stimulant sur le métabolisme, car elles favorisent la synthèse d'enzymes hydrolysantes . Les GAs agissent également sur la teneur en auxines qui est souvent augmentée soit par stimulation des synthèses soit par inhibition des auxines-oxydases,
- une action complexe sur les processus impliqués dans la floraison; elles seraient plutôt inhibitrices de l'induction florale en particulier chez les arbres fruitiers. Par contre, pour des espèces ayant besoin de froid pour fleurir, une application de GA₃ pourrait permettre la floraison en absence de froid,
- une action également complexe sur les phénomènes de levée de dormance ,
- enfin , dans le domaine de l'organogenèse, les GAs se comportent en antagonistes .

Les cytokinines sont des adénines substituées dérivant des purines.

Les ARN de transfert constituent une source potentielle de CKs chez les végétaux, mais cette voie ne serait cependant pas la seule puisqu'il est admis aujourd'hui,que la voie privilégiée de biosynthèse de CKs provient du métabolisme de l'AMV. Dans les deux voies proposées, l'IPP joue un rôle stratégique . Dans ce cas, il se condense avec une adénosine-5'-monophosphate, alors que dans l'autre cas, il se condense avec un résidu adénine issu des ARN de transfert .

Les CKs sont très actives et présentent de nombreuses actions dont les principales sont:
- un effet très net sur la division cellulaire;
- un rôle très net également dans l'organogenèse où elles sont antagonistes de la rhizogenèse ,
- une action stimulante sur le métabolisme, d'une part en favorisant les synthèses protéiques et d'autre part, en protégeant les métabolites contre les actions des enzymes hydrolysantes . Cet effet provoque un retard de sénescence au point que des feuilles traitées avec des CKs peuvent entrer en concurrence avec des feuilles plus jeunes au niveau des corrélations morphogènes de la plante,
- un effet antagoniste de la dominance apicale; des bourgeons axillaires traités par des CKs entrent en croissance et en compétition avec l'axe terminal.

L'ABA est synthétisé à partir de l'AMV via le farnésyl pyrophosphate (FPP) ( MOORE T.C. Biochemistry and physiology of plant hormones ed: Springer Verlag, New York Heidelberg, Berlin, 1979 ) . Une autre voie menant à la synthèse de cet inhibiteur a également été décrite. Elle dérive des caroténoïdes et en particulier, de la violaxanthine.

Les sites de synthèse de l'ABA dans la plante sont multiples . En effet , lorsque des feuilles isolées, des tiges ou des racines sont placées en condition de "stress" hydrique, une augmentation du taux d'ABA est observée .

L'ABA est également présent dans les fruits et dans les graines .

L'ABA joue un rôle majeur dans la régulation de la fermeture des stomates.

L'ABA joue également un rôle dans les phénomènes de dormance observés au niveau de bourgeons ou de graines. Il exerce aussi une action complexe sur la floraison. Il semble que l'ABA soit très impliqué dans le contrôle de l'abscission des fruits.

La position clé occupée par le FPP dans les voies de biosynthèse des GAs et de l'ABA d'une part et, d'autre part, la nature isoprènique des groupements substituants des CKs naturelles montrent toute l'importance de la MK dans la biosynthèse de ces différents types de régulateurs de croissance.

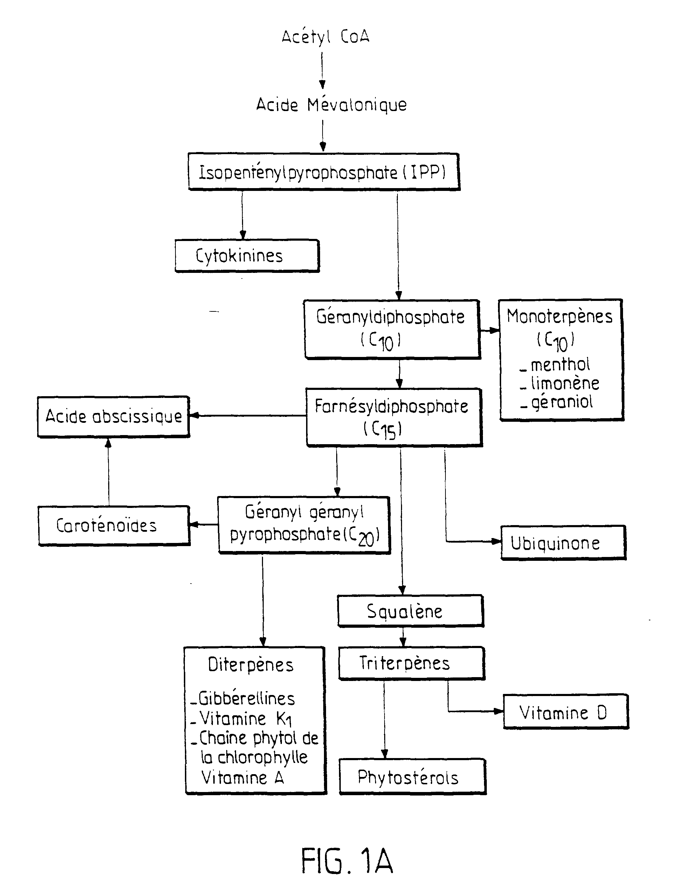

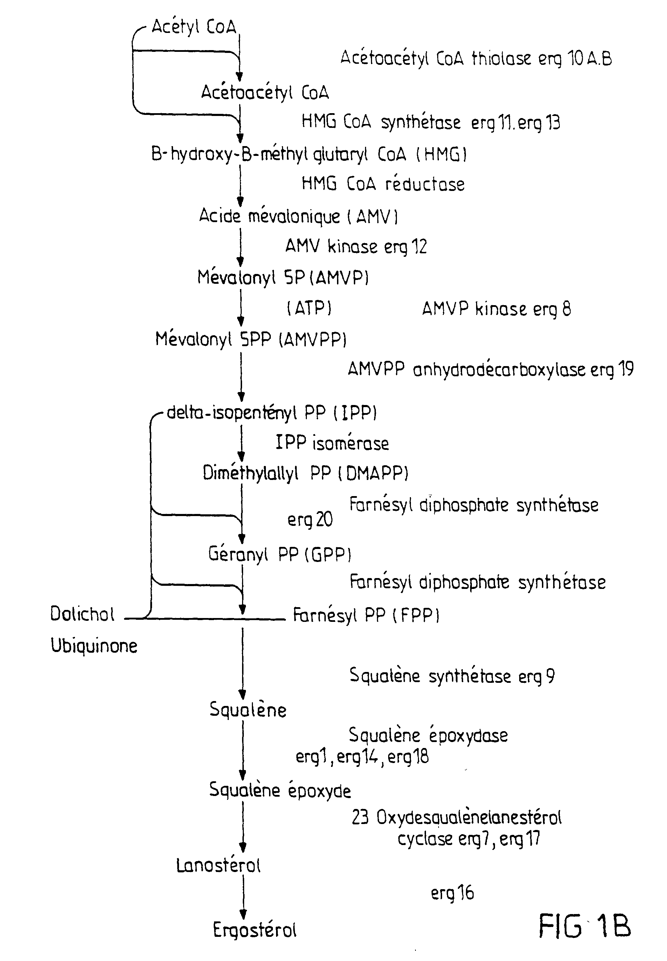

Ces voies sont résumées sur les diagrames des figures 1A et 1B .

Des plantes dans lesquelles ont été intégrés des gènes codant pour des enzymes intervenant dans la voie de biosynthèse des phytostérols ont déjà été décrites.

La demande Internationale WO-9 113 078 ( AMOCO ) décrit des plantes transgéniques portant dans leur génome des fragments d'ADN codant pour la géranyle-géranyle pyrophosphate synthase (GGPP).

Cette étape , dans la boie de biosynthèse des caroténoïdes est située en aval par rapport aux étapes précoces de la voie de biosynthèse des stérols.

Un compte-rendu de l'"Annual Meeting of the American Society of Plant Physiologists" ( Albuquerque, Nouveau Mexique, 28 Juillet - 1er Août 1991) , concerne des plantes dans lesquelles a été intégré un gène de hamster codant pour la 3-hydroxy-3-méthylglutaryle co-enzyme A réductase (HMGR).

On notera que cette étape est en amont de l'étape de transformation de l'acide mévalonique, dans laquelle la HMGR n'intervient pas.

La demande EP-0.480.730 concerne des plantes dans lesquelles a été intégré un gène de hamster codant pour la 3-hydroxy-3-méthylglutaryle co-enzyme A réductase (HMGR).

Cette enzyme n'intervient pas dans la chaîne de réaction biochimique par laquelle l'acide mévalonique est transformé en farnésyl diphosphate.

Cette demande appartient à l'état de la technique visé par l'article 54(3) CBE.

Des gènes de nature différente ont aussi déjà été insérés dans diverses plantes au moyen principalement de dérivés de plasmides Ti. Cette technique qui a notamment été décrite par KLEE et al. (Ann. Rev., Plant Physiol 38, 1987, 467-486), permet l'introduction d'un nombre limité de gènes en une seule opération.

Néanmoins, cette méthode très récente reste inapplicable pour différentes espèces ou variétés, en particulier les graminées.

En outre,même si l'insertion du gène est rendue possible, il est nécessaire qu'il soit exprimé afin de produire ses effets dans les cellules végétales. Ces techniques bien qu'évoluant rapidement, ne permettent pas d'introduire n'importe quel gène dans n'importe qu'elle cellule et de nombreux échecs d'expression de gènes étrangers à la plante ont été enregistrés par diverses équipes.

Outre les problèmes d'insertion des gènes et la fabrication d'un vecteur adéquat, on peut être confronté à des problèmes d'expression des gènes transférés dans les cellules végétales aboutissant à une absence d'expression ou une très faible expression et à une activité inexistante du produit du gène recherché. Ces problèmes d'expression peuvent être dus notamment à l'incapacité du promoteur, qui est un des facteurs intervenant dans la régulation de l'expression du gène, à induire la synthèse d'ARN messager.

Il peut aussi s'agir d'une incapacité de la plante à modifier de manière adéquate le produit de traduction de l'ARN messager .

Un autre problème réside dans les difficultés rencontrées dans la régénération de certaines espèces ou variétés ou même dans l'impossibilité à effectuer une telle opération . Certaines espèces de légumineuses et de graminées sont récalcitrantes à la régénération par des méthodes classiques .

Ainsi , l'analyse de l'état de la technique met en évidence que , malgré les résultats intéressants obtenus ces dernières années dans le domaine de la biologie moléculaire végétale , de nombreux obstacles s'opposent au transfert de gènes et à l'expression de ces gènes dans des espèces ou des variétés spécifiques de plantes présentant des intérêts agronomiques

La demanderesse s'est donc attachée à trouver des moyens permettant d'insérer dans des plantes d'intérêt agronomique des gènes codant pour les enzymes des étapes précoces des voies de la biosynthèse des phytostérols et permettant l'expression desdits gènes dans les plantes.

La demanderesse s'est d'autre part attachée à mettre au point un procédé permettant de modifier l'équilibre hormonal endogène des plantes afin d'améliorer la capacité de régénération des espèces difficiles à régénérer .

La demanderesse a ainsi montré d'une manière surprenante qu'il est possible d'insérer de manière stable dans le génome de ces plantes , des gènes codant pour les voies de biosynthèse des phytostérols , et que les plantes ainsi obtenues présentent de nombreux avantages, tels qu'un développement plus important, une productivité améliorée et une amélioration de leur capacité à régénérer.

La présente invention a donc pour objet une plante caractérisée en ce qu'elle porte dans son génome un ou plusieurs gènes codant pour une ou plusieurs enzymes des étapes précoces de la voie de biosynthèse des phytostérols constituées par la chaine de réactions biochimiques par laquelle l'acide mévalonique est transformée en farnésyl diphosphate, lesdits gènes soit ne se retrouvant pas dans la plante native, soit étant originaires de ladite plante , auquel cas ils sont présents en un nombre de copies supérieur à celui de la plante native ou ils sont modifiés dans leur structure ou leur fonction par rapport à ceux de la plante native .

On entend, dans la présente demande , par étapes précoces de la voie de biosynthèse des phytostérols , la chaîne de réaction biochimique par laquelle l'acide mévalonique est, comme indiqué sur les figures 1A et 1B, transformé en farnésyl diphosphate.

Avantageusement , l'étape pour laquelle codent ce ou ces gènes est la transformation de l'acide mévalonique en mévalonyl 5-phosphate . Dans ce cas, la plante peut porter et/ou exprimer un ou plusieurs gènes codant pour une ou plusieurs mévalonate kinases ( MK) , et en particulier le gène ERG12 , ou l'un de ses dérivés ou fragments exprimant une activité mévalonate kinase . Ce gène a été isolé chez la levure par Oulmouden et Karst ( Gene , 88, 253-257, 1990 ) et séquencé par les mêmes auteurs ( Curr Genet , 19, 9-14, 1991).

Il peut notamment s'agir de gènes codant pour l'activité mévalonate kinase chez les crucifères et en particulier chez Arabidopsis ou chez le colza.

Lesdites plantes peuvent porter aussi un ou plusieurs gènes codant pour une farnésyl diphosphate synthétase, tels que le gène ERG 20 de levure (Chambon et al., Curr. Genet, 18, 41, 1990 ) ou l'un de ses dérivés ou fragments exprimant une activité farnésyl diphosphate synthétase.

Elles peuvent également porter un ou plusieurs gènes codant pour une AMVP kinase ( mévalonyl 5-P-kinase), tels que le gène ERG 8 ( Tsay et al., Mol. Cell. Biol., 11, n°2, 620, 1991) ou l'un de ses dérivés ou fragments exprimant une activité AMVP-kinase.

Les étapes des voies de biosynthèse dans lesquelles interviennent la farnésyl diphosphate synthétase et l'AMVP-kinase sont respectivement , comme indiqué sur la figure 1B, les étapes de transformation du diméthylallyl pyrophosphate (DMAPP) en farnésyl pyrophosphate (FPP) et de transformation du mévalonyl 5-phosphate (AMVP) en mévalonyl 5-pyrophosphate (AMVPP).

Le ou les gènes peuvent avoir été obtenus par clonage à partir d'ADN extrait d'organismes ou de virus vivants ou de toute autre matière biologique , ou peuvent avoir été synthétisés.

Lesdites plantes, portant ces gènes codant pour un ou plusieurs enzymes des étapes précoces de la voie de biosynthèse des phytostérols , sont avantageusement des plantes appartenant à la catégorie des plantes oléoprotéagineuses , et en particulier de la famille des crucifères telle que l'espèce colza ou de la famille des Fabacées telle que l'espèce soja .

Ladite plante peut aussi être une plante des espèces tournesol , aubergine ( Solanum melongena var.) ou Vigna radiata.

De manière avantageuse, le ou les gènes sont placés sous le contrôle d'un promoteur inséré à proximité du ou des gènes, de manière à permettre l'expression de leurs produits biologiquement actifs .

La présente invention a d'autre part pour objet un vecteur pour l'insertion d'au moins un gène précité dans des cellules végétales, caractérisé en ce qu'il comprend :
- la séquence du gène à insérer ,
- une ou plusieurs séquences du type promoteur en amont du gène à insérer ,
- une ou plusieurs séquences du type terminateur en aval du gène à insérer ,
- un ou plusieurs marqueurs permettant la sélection des cellules végétales dans lequel le gène s'est inséré, et
- des séquences d'un plasmide Ti permettant le transfert desdits gènes et marqueurs d'une bactérie vers une cellule végétale , lesdits gènes et marqueurs étant orientés et insérés de manière à permettre leur expression.

Préférentiellement , le gène à insérer code pour une ou plusieurs enzymes des étapes précoces de la voie de biosynthèse des phytostérols . Ce peut-être un gène codant pour la mévalonate kinase et en particulier le gène ERG12,précédemment cité .

Les marqueurs permettant la sélection des cellules dans lesquelles le ou les gènes se sont insérés peuvent être toute activité enzymatique permettant la sélection directe ou indirecte des cellules dans lesquelles il y a eu insertion, et en particulier des marqueurs de résistance à des antibiotiques tels que le marqueur de résistance à la kanamycine chez les végétaux , ou des marqueurs dont l'activité enzymatique est utilisée pour colorer les cellules végétales tels que le gène lacZ.

Un tel vecteur d'insertion est notamment , le plasmide pFAB2 portant le gène ERG12 déposé sous le n° I-1176 du 14 Février 1992 auprès de la Collection Nationale de Culture des Microorganismes.

Un vecteur peut être aussi un dérivé de ce plasmide et dans lequel le gène ERG12 a été remplacé par un autre gène codant pour une ou plusieurs des enzymes de la voie de biosynthèse des stérols .

Un autre objet de la présente invention est un procédé de traitement des plantes destiné à améliorer leur productivité , et en particulier leur performance de croissance ou leur précocité de floraison , par insertion dans leur génome d'un ou plusieurs gènes codant pour une ou plusieurs enzymes des étapes précoces de la voie de synthèse des phytostérols .

L'insertion du ou des gènes est préférentiellement effectuée par transfert dans des cellules individualisées ou dans des groupes de cellules d'un vecteur tel que décrit précédemment , éventuellement en présence d'un autre vecteur de type plasmide Ti ayant des fonctions permettant ce transfert.

Préférentiellement , de tels vecteurs seront dans des souches d'Agrobactérium .

Le transfert peut aussi être effectué , en absence de vecteurs ayant de telles fonctions , par une technique physique appropriée telle que l'électroporation.

Le transfert du vecteur à partir d'Agrobactérium tumefasciens dans des cellules végétales est effectué par toute méthode connue en soi par l'homme du métier et avantageusement par inoculation de disques foliaires.

Les cellules ainsi inoculées sont alors sélectionnées pour la présence des vecteurs et les plantes sont régénérées par les méthodes connues en soi et notamment décrites dans "Plant molecular physiology manual " ( Gelvin et Schilperdort, Kluwers Academic Publishers , 1988 ). Selon la présente invention, on a obtenu de bons résultats en transférant le gène ERG12 de levure par le système Agrobacterium.

La présente invention est illustrée par les exemples qui suivent avec référence aux dessins annexés , dans lesquels:
- Les figures 1A et 1B , déjà citées , sont des diagrammes illustratifs de données connues ;

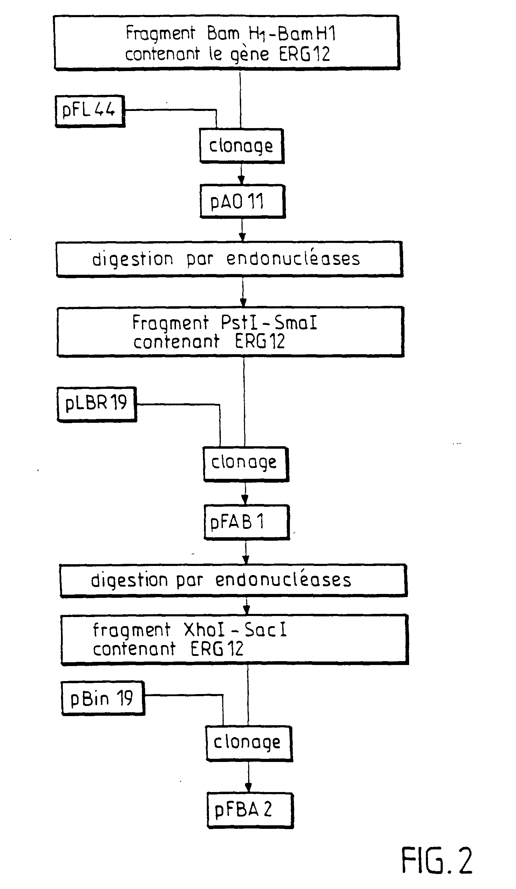

- la figure 2 est un diagrame résumant le procédé d'obtention du plasmide pFAB2 portant le gène ERG12.
- la figure 3 est une carte de restriction partielle du fragment BamHI- BamH-I de 2,8 kilobases (kb) contenant le gène ERG12.
- les figures 4 et 5 sont des cartes de restriction respectivement des plasmides et pLBR19 et pFL44 .
- la figure 6 résume l'obtention du plasmide pFAB1 à partir du plasmide pLBR19 par insertion du fragment BamH I-BamHI contenant le gène ERG12 .
- la figure 7 est la photographie d'un gel d'électrophorèse des produits d'hydrolyse de pAO11 par BamH-l (piste 1 ), de pFAB1 par BamHl (piste 2), de pLBR19 par Smal (piste 3), de pLBR19 par Pstl ( piste 4) , de pAO11 par Pstl et Smal ( piste 5) et de l'ADN témoin du bactériophage lambda hydrolysé par Hind-III. Les deux flèches à gauche de la photographie, correspondent respectivement aux positions des bandes de 1,8 kb et 0,8 kb .
- la figure 8 représente le carte du plasmide pBinl9 .
- la figure 9 représente l'obtention du plasmide pFAB2 à partir des plasmides pBin19 et pFAB1.
- la figure 10 est une photographie d'un gel d'électrophorèse coloré par du bromure d'éthidium et révélé sous rayons ultraviolets d'ADN du bactériophage lambda hydrolysé par Hind III ( piste 1 ) , et des produits d'hydrolyse de pFAB1 par BamH1 ( piste 2), de pFAB2 par BamH1 (piste 3 ), de pBin19 par Sall ( piste 4), de pBin19 par SacI (piste 5), et de pFAB1 par Xhol et Sacl ( piste 6). Les flèches à droite de la photographie indiquent les positions respectives des bandes de taille 1,8 kb et 0,8 kb.
- les figures 11 et 12 correspondent à des diagrammes en chromatographie sur couche mince de gel de silice, de surnageants de culture de la souche d'Escherichia coli 71/18 portant respectivement les plasmides pFL44 (figure 11A), pAO11 ( figure 11B) et pFAB1 ( figure 11C) et de la souche d'Agrobacterium tumefasciens LBA4404 portant respectivement les plasmides pBin19 (figure 12A) et pFAB2 (figure 12B). Les abréviations AMV et AMVP sur les figures , correspondent respectivement à la position des pics de l'acide mévalonique et au mévalonyl 5 P.
- la figure 13 correspond à un autoradiogramme d'un Southern blot dont l'hybridation a été effectuée par une sonde portant le gène ERG12. Les pistes 1, 2 et 3 correspondent respectivement aux plasmides pAO11 ( témoin positif ), à l'ADN total extrait d'une plante " témoin H₂O" , et à l'ADN total extrait d'une plante transformée .
- la figure 14 est la séquence partielle du gène ERG12, les localisations des oligonucléotides ON₁ et ON₂ et du site de restriction BamHI étant indiquées sur cette figure .

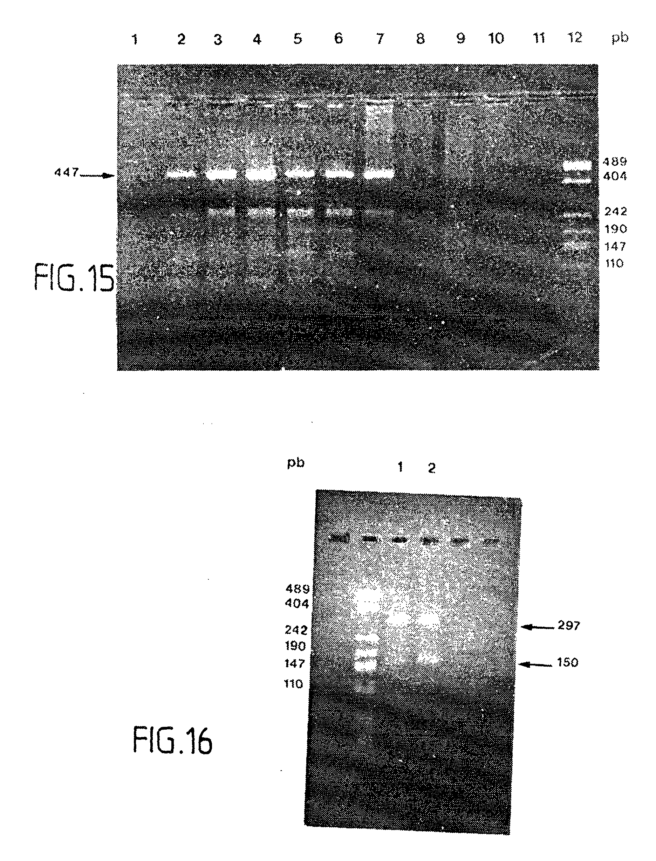

- la figure 15 est un gel d'électrophorèse de produits de réaction d'amplification par la technique PCR . Les pistes 1 à 6 correspondent à l'amplification à partir d'ADN total extrait de tabacs transformés , la piste 7 correspond à l'amplification à partir de l'ADN génomique de levure , la piste 8 correspond à l'amplification avec l'amorce ON1 seule , la piste 9 correspond à l'amplification avec l'amorce ON2 seule , la piste 10 correspond à l'amplification à partir de l'ADN total extrait d'un tabac " témoin H₂O" , la piste 11 correspond à un témoin de non-contamination ( eau stérile) et la piste 12 correspond au plasmide pBlueScript hydrolysé par HpaII (marqueur de poids moléculaire ). La taille des bandes d'hydrolyse du marqueur est indiquée à droite de la photographie .
- La figure 16 est une photographie d'un gel d'agarose après amplification par la technique PCR et hydrolyse par BamH1 respectivement d'ADN total extrait d'une plante transformée ( piste 1 ) et d'ADN génomique de levure (piste 2). La piste non marquée à gauche de la piste 1 est de l'ADN du plasmide pBluescript hydrolysé par HpaII .

### EXEMPLES

### Matériel et méthodes utilisés dans les exemples .

### I. Techniques de biologie moléculaire.

### 1. Matériel biologique

### 1.1- Souches bactériennes.

Les souches bactériennes utilisées soit comme source de vecteur, soit comme souche hôte des plasmides recombinants, dérivent d'Escherichia coli K12. La souche Hb101 (BOYER et ROULLAND-DUSSOIX - J. Mol. Biol. 41, 1969, 469-472 ), résistante à la streptomycine a été fournie par le Laboratoire de biologie de la rhizosphère de l'Institut National de la Recherche Agronomique ( INRA ) de Versailles et la souche 71/18 (MESSING et al. Proc. Natl. Acad. Sci. USA, 74, 1977,3642-3646 ) par le Laboratoire de Biochimie et Génétique des Microorganismes de l'Université de Poitiers.

La souche HB101 utilisée pour la mobilisation du vecteur de transfert vers A.tumefasciens contient le plasmide pRK2013 qui apporte les résistances à la kanamycine et à la streptomycine (BEVAN, Nucleic Acids Research ,12, 8711-8721 1984 ).

La souche d'A. tumefasciens provient également du Laboratoire de Biologie de la Rhizosphère de l'Institut National de la Recherche Agronomique (INRA ) de Versailles . Il s'agit de la souche LBA4404 (HOEKMA et al., Nature , 303,179-180 1983), résistante à la streptomycine et à la rifampicine. Elle dérive de la souche Ach5 et possède un plasmide Ti délété de la région T. Elle contient en plus le vecteur binaire de transfert Bin 19 (BEVAN, 1984, précédemment cité ) .

Les souches d'E. coli sont cultivées à 37°C et A. tumefasciens est cultivé à 28°C.

### 1.2. Les plasmides .

Trois plasmides ont été utilisés pour la construction du vecteur de transfert . Ils sont décrits ci-dessous :
- Plasmide pAO11 : obtenu par clonage d'un fragment BamH I-BamH I de 2,8 kb contenant le gène ERG12 de levure, dans le plasmide pFL44
- Plasmide pLBR19 : il présente l'avantage de posséder de nombreux sites uniques de restriction insérés entre les séquences promoteur et terminateur de l'ARN 35S du virus de la mosaïque du chou-fleur . Ce plasmide comporte également un gène de résistance à l'ampicilline . Il a été fourni par le Laboratoire de Biologie de la rhizosphère de l'INRA de Versailles.
- Plasmide pBin19 : ce vecteur autonome, capable de se répliquer dans E.coli et dans A.tumefasciens, dérive du plasmide pRK252 modifié par insertion d'un gène de résistance à la kanamycine provenant de Streptococcus faecalis ( BEVAN, 1984, précédemment cité). Il comporte les séquences bordures droite et gauche de l'ADN-T du plasmide pTi37.

Ont été insérés entre ces deux séquences :
(1) un fragment d'ADN de 440 pb provenant du phage M13 et qui contient la région de complémentation de la β-galactosidase (Lac Z) dans laquelle a été insérée une séquence qui porte plusieurs sites de clonage.
(2) un gène chimérique conférant une résistance à la kanamycine chez les végétaux, construit par insertion de la région codante d'une aminoglycoside transférase de type II (provenant de Tn5) entre les séquences régulatrices du gène de la nopaline synthétase.

Toute séquence d'ADN insérée au niveau du site de clonage multiple, compris dans la région délimitée par les séquences bordures droite et gauche de l'ADN-T, peut donc être transférée et intégrée dans le génome d'une plante, le transfert étant induit , à distance , par les fonctions de virulence portées par un plasmide Ti hébergé par la même souche d'A.tumefasciens . Le système de sélection est assuré par la co-intégration du gène de résistance à la kanamycine.

Les plasmides pAO11 et pFL44 ont été fournis par le Laboratoire de Biochimie et de Génétique des Microorganismes de l'Université de Poitiers , tandis que le plasmide pLBR19 a été fourni par le Laboratoire de Biologie de la Rhizosphère de l'Institut National de la Recherche Agronomique ( INRA ) de Versailles .

### 2. Milieux de culture.

- Milieu L. Broth , utilisé pour la culture des souches d'E.coli :
   - Tryptone (Biokar) 10 g
   - Extrait de levure (Biokar) 5 g
   - NaCl 5 g
   - Eau distillée 11

Le pH du milieu est amené à 7 avant stérilisation (120°C pendant 20 min) . Ce milieu est utilisé liquide ou solidifié par de l'agar à 20 g/l . La gélose molle L.Broth utilisée dans les expériences de transformation est obtenue par addition d'agar à la concentration de 8 g/l .
- Milieu AP, utilisé pour la culture des agrobactéries :
   - Extrait de levure (Biokar) 5 g
   - Hydrolysat de caséine ( Sigma) 0,5 g
   - Mannitol 8 g
   - (NH₄)₂SO₄ 2 g
   - NaCl 5 g
   - Eau distillée 1 litre

Le pH du milieu est amené à 6,6 avant stérilisation (115°C pendant 20 min). Ce milieu est utilisé liquide ou solidifié par de l'agar à la concentration de 15 g/l.

Les antibiotiques sont utilisés aux concentrations suivantes (mg.l⁻¹):

| | E. coli | A.tumefasciens |
|---|---|---|
| ampicilline | 100 | - |
| kanamycine | 50 | 50 |
| rifampicine | - | 50 |
| streptomycine | - | 500 |

Des stocks concentrés de chaque antibiotique sont conservés à -20°C. L'antibiotique est rajouté au milieu fondu, refroidi à 50°C, au moment de couler les boîtes.

### 3- Préparation et transformation de cellules compétentes d'E.coli.

### 3.1 - Préparation des cellules compétentes.

Une préculture d'une nuit de la souche d'E.coli est réalisée en milieu LB à partir d'une colonie isolée sur boîte. 500 ml de milieu LB sont inoculés avec 4 ml de cette préculture . Lorsque la densité optique à 600 nm atteint la valeur de 0,2, la culture est placée 15 à 20 min dans la glace puis centrifugée 10 min à 6000 rpm ( rotor Kontron A8.24) à 4°C. Le culot est mis en suspension dans 200 ml de CaCl₂ 100 mM.Le culot est repris dans 5 ml de CaCl₂ 100 mM et placé dans la glace durant une nuit.

Les bactéries compétentes ainsi obtenues sont stockées en fractions de 1 ml en présence de glycérol à 30%. Elles peuvent ainsi se conserver plusieurs mois à -80°C.

### 3.2.- Transformation.

Les cellules compétentes sont décongelées ; 200 ml de suspension sont mis en présence de 0,1 à 1 *µ*g d'ADN pendant 30 min dans la glace . Le mélange est ensuite soumis à un choc thermique par passage de 2 min à 42°C. Après addition de 1 ml de LB liquide, le mélange est incubé pendant une heure à 37°C. L'ensemble du milieu de transformation est mélangé à 3 ml de gélose molle maintenue à 45°C puis étalé sur milieu sélectif (milieu LB contenant les antibiotiques appropriés ) . Les clones résistants sont subclonés et identifiés.

### 3.3- Identification des colonies bactériennes contenant les plasmides recombinants.

On réalise des minipréparations d'ADN plasmidique à partir d'un certain nombre de clones transformés . L'ADN de chaque clone est digéré par les enzymes de restriction appropriées . Les fragments obtenus sont analysés par électrophorèse sur gel d'agarose, par rapport à un vecteur témoin. Ceci permet d'identifier les clones bactériens qui contiennent le plasmide recombinant.

### 4. Mobilisation E.coli - A. tumefasciens par conjugaison triparentale.

Le vecteur de transfert dans lequel est inséré le gène chimérique (plasmide Binl9) n'est pas directement transférable de E.coli à A.tumefasciens mais peut être mobilisé si les fonctions de transfert et de mobilisation sont apportées par un plasmide dit "mobilisateur ". Il s'agit ici du plasmide pRK2O13. Ce plasmide porte les fonctions de transfert du plasmide RK2 , le gène mob du plasmide ColEl et l'origine de réplication de ce même plasmide qui ne lui permet pas de se répliquer dans A.tumefasciens .

Les conjugaisons triparentales mettant en présence la souche d'E.coli donatrice, la souche d'A.tumefasciens réceptrice et la souche d'E.coli possédant le plasmide pRK2013, sont réalisées.

Chaque souche est cultivée dans 5 ml du milieu de culture adéquat contenant les antibiotiques appropriés, pendant une nuit . Les cultures sont centrifugées 5 min à 6000 rpm (Kontron A8.24) à 4°C . Les culots sont remis en suspension dans du milieu sans antibiotique puis centrifugés à nouveau . Chaque culot est finalement repris par 5 ml de milieu de culture.

On dépose ensuite 1 ml de chacune des suspensions sur un filtre de porosité 0,45 *µ*m (Millipore, type HA). Le filtre est placé sur milieu AP gélosé sans antibiotique dans une boîte de Pétri de diamètre 55 mm et incubé une nuit à 28°C.

Les bactéries sont récupérées sur le filtre et remises en suspension dans 0,5 ml de milieu AP. Des dilutions de cette suspension sont effectuées et 150 à 200 *µ*l de chaque dilution sont étalés sur milieu AP sélectif . Les boîtes sont placées à 28°C . Au bout de 3 à 5 jours , les clones apparaissent .

### 5- Extraction des acides nucléiques.

### 5.1 - Extraction de plasmides d'E.coli

### a- minipréparation.

Le protocole suivant a donné les meilleurs résultats, de façon reproductible.

1,5 ml de milieu LB contenant les antibiotiques appropriés sont ensemencés à partir d'une colonie dans un tube à essai. Le tube est ensuite placé à 37°C, sous agitation,durant une nuit. La culture est transférée dans un tube Eppendorf et centrifugée . Le culot est repris par 150*µ*l de STETL (saccharose 8%, Triton X100 5%, EDTA 50 mM, Tris 50 mM pH8, Lysozyme 0,5 mg/l). Le mélange est placé dans un bain-marie bouillant pendant 1 min, puis centrifugé 15 min. L'ADN chromosomique dénaturé , les protéines et les débris cellulaires constituent un culot qui est éliminé. L'ADN du surnageant est précipité par addition de 150 *µ*l d'isopropanol pendant 15 min à -20°C. Après centrifugation, le culot est séché puis repris par 120*µ*l de TE 10/1 (Tris 10mM, EDTA 1mM pH 7,5). Deux extractions au phénol-chloroforme (3v:lv) sont effectuées . L'ADN contenu dans la phase aqueuse est précipité par addition d'un dixième de volume d'acétate de sodium 3M et de deux volumes d'éthanol absolu. Après lavage à l'éthanol 80%, le culot est séché puis repris par 20 *µ*l de TE 10/1. 5 à 10 *µ*l de l'ADN préparé sont suffisants pour une analyse éventuelle, la quantité d'ADN pouvant varier en fonction de la taille du plasmide et de la souche bactérienne.

### b- maxipréparation.

Après amplification au chloramphénicol (MANIATIS-Molecular Cloning - A Laboratory Manual Cold Harbor Laboratory, Cold Spring Harbor , New York,1982), une culture bactérienne réalisée dans un litre de milieu LB liquide contenant les antibiotiques appropriés est centrifugée à 6000 rpm pendant 10 min (rotor Kontron A6.14), à 4°C. Les cellules récoltées sont lavées dans du TE 10/1 pH 8. Le culot est repris par 11,4 ml de TE 200/100 (Tris 200 mM, EDTA 100 mM pH 8) additionné de 0,6 ml de lysozyme à 20 mg.ml. Le mélange est laissé à température ambiante pendant 10 min. 4 ml de TE 200/100 pH 8 contenant 6% ( p/v) de sarkosyl sont ajoutés et le tube est soumis à une agitation douce pendant 10 min à température ambiante. Les débris cellulaires sont éliminés par centrifugation à 12000 rpm pendant 30 min. Pour 7,6 ml de lysat récupéré, on ajoute 8 g de chlorure de césium (CsCl) et 1 ml de bromure d'éthidium (BET) à 10 mg/ml . Une ultracentrifugation est ensuite réalisée pendant 36 heures à 42000 rpm ( rotor Beckman 50 Ti), à 20°C . A l'issue de cette centrifugation, les tubes sont éclairés en lumière UV (366 nm). Deux bandes fluorescentes sont observées :
- une bande supérieure constituée d'ADN chromosomique et d'ADN plasmidique ouvert,
- une bande inférieure constituée d'ADN plasmidique fermé (superenroulé).

La bande inférieure est récupérée soit à l'aide d'une seringue munie d'une aiguille, soit à l'aide d'une pompe péristaltique munie d'un cathéter . Le BET est extrait avec de l'isobutanol saturé en TE 10/1 pH 7,5. Une dialyse est ensuite réalisée contre du TE 10/1 pH 7,5 pendant environ 24 heures . Finalement, l'ADN plasmidique est précipité par addition d'un dixième de volume d'acétate de sodium 3M et de deux volumes d'éthanol absolu. La concentration de la solution est estimée par spectrophotométrie.

### 5.2 - Extraction de l'ADN végétal total.

La méthode employée est décrite par ROGERS et BENDICH ( Plant Mol. Biol. 5,1985, 69-76). Elle est basée sur l'utilisation de bromure de cétyltriméthylammonium (CTAB). Cette technique permet d'obtenir, chez le tabac, environ 20 *µ*g d'ADN en partant de 500 mg de feuilles .

500 mg de feuilles jeunes sont broyées dans un mortier avec de l'azote liquide . Le broyat est transféré dans un tube Eppendorf . Un volume de 2 X CTAB à 65°C [ CTAB 2% (p/v), Tris 100 mM pH 8, EDTA 20 mM pH 8, NaCl 1,4 M, PVP 1% (p/v) ] est ajouté au broyat. Le mélange est incubé 5 min à 65°C et reçoit ensuite un volume de chloroforme-alcool isoamylique (24 v : 1v). Après homogénéisation et centrifuga- tion 5 min à 11000 rpm, la phase supérieure est récupérée et transférée dans un nouveau tube . On ajoute alors un dixième de volume de CTAB 10% à 65°C [CTAB 10% (p/v), NaCl 0,7 M ] , on homogénéise et on centrifuge de nouveau. La phase supérieure est récupérée et additionnée d'un volume de tampon CTAB de précipitation à 65°C [CTAB 1 % (p/v), Tris 50 mM pH 8, EDTA 10 mM pH 8 ] . Après homogénéisation et centrifugation,le culot contenant les acides nucléiques est repris par 300 *µ*l de TE high ( Tris 10 mM pH 8, EDTA 1 mM pH 8, NaCl 1M) et maintenu à 65°C pendant 10 min. L'ADN est précipité par addition de 600 *µ*l d'éthanol absolu à -20°C pendant une nuit. Le lendemain, le culot est lavé dans l'éthanol à 80%, séché et repris par 20 *µ*l de 0,1 X TE ( Tris 1 mM pH 8, EDTA 1 mM pH 8).

### 6. Digestion de l'ADN par des endonucléases de restriction.

Les enzymes de restriction sont utilisées conformément aux indications données par le fournisseur (GIBCO). La composition du tampon d'incubation est fonction de l'enzyme utilisée de même que la température d'incubation (pour la plupart des enzymes, cette température est de 37°C).

Le nombre d'unités enzymatiques utilisées dépend de l'ADN à digérer et de l'enzyme utilisée . Des incubations de 1 à 2 heures avec 5 à 10 unités d'enzyme par *µ*g d'ADN, pour l'ADN plasmidique et d'une nuit, pour l'ADN génomique sont généralement conseillées Les ADNs obtenus par minipréparation sont traités par la RNase A préparée selon MANIATIS ( 1982 précédemment cité ) . Les digestions sont arrêtées par un passage de 10 min à 65°C.

### 7. Electrophorèse horizontale de l'ADN en gel d'agarose .

On utilise des gels d'agarose dont la concentration varie de 0,8 à 3 % ( p/v) , selon la taille des fragments à séparer . L'agarose ( Appligène, qualité biologie molé- culaire) est mélangé au volume requis de tampon TAE ( Tris-acétate 0,04 M, EDTA 0,002 M pH 8) et porté à ébullition au four à microondes . Il est refroidi jusqu'à 60°C avant d'être coulé dans la cuve d'électrophorèse.

Lorsque les digestions ont eu lieu dans un volume supérieur à 20 *µ*l, l'ADN est précipité et le culot, après rinçage à l'éthanol 80% , est repris dans 15 *µ*l de TE 10/1 pH 7,5 . Les échantillons sont additionnés d'un mélange [ 50% glycérol, EDTA 0,1 M, Bleu de bromophénol 0,1 % , xylène cyanol 0,1% ] pour environ un cinquième de leur volume . Ceci permet de suivre la migration.

La migration s'effectue à 20°C de la cathode vers l'anode , sous une tension constante de 2 à 10 V/cm. La coloration des gels au BET ( 10 mg/ml ) est effectuée en même temps que la migration. Le gel est observé et photographié sous lumière UV ( 254 nm ). Les masses moléculaires des fragments d'ADN sont estimées par comparaison avec les fragments de l'ADN du bactériophage lambda digéré par Hind III ( Boehringer).

### 8. Elution d'un fragment d'ADN d'un gel d'agarose.

Après électrophorèse, la purification de fragments d'ADN peut être réalisée en découpant sous UV, la bande d'agarose contenant le fragment à purifier. Deux techniques d'élution ont été utilisées.

La première technique utilisée consiste en une électroélution de l'ADN à l'aide d'un Biotrap ( commercialisé par Schleicher et Schuell). La bande intéressante est placée dans le compartiment central du Biotrap rempli auparavant de tampon TAE. L'électroélution se déroule sous une différence de potentiel de 150 V durant 1 à 2 heures et peut être suivie en lumière UV. Après une inversion du courant de 20 secondes , la solution d'ADN peut être récupérée au niveau du réservoir délimité par deux membranes dont une retient l'ADN . Cette inversion du courant permet de libérer l'ADN retenu par la membrane . L'ADN est ensuite précipité à -20°C pendant 30 min après addition de 2 volumes d'éthanol absolu et d'un dixième de volume d'acétate de sodium 3 M. Le précipité est récupéré par centrifugation à 12000 rpm pendant 15 min, rincé avec de l'éthanol 80%. Le culot est ensuite séché et remis en suspension dans du TE 10/1 pH 7,5.

La seconde technique utilisée est celle du "freeze-squeeze" amélioré ( TAUTZ et RENZ, Anal. Biochem.132,14-19(1983)). Les parties du gel renfermant les bandes intéressantes sont découpées et équilibrées pendant 30 min à l'obscurité à 4°C contre 10 volumes de tampon TEN ( Tris 10 mM pH 7,1, EDTA 1 mM, NaCl 0,3 M). Chaque fragment de gel est alors transféré dans un tube Eppendorf percé au fond et obstrué par de la laine de verre siliconée . Ce tube est lui-même introduit dans un autre tube Eppendorf . Le tout est congelé à -80°C, puis centrifugé 15 min à 12000 rpm. L'ADN est ensuite précipité et traité comme précédemment . Cette technique est beaucoup plus rapide que la technique d'électroélution utilisant le Biotrap et permet d'obtenir un rendement supérieur .

### 9. Ligatures des fragments de restriction par l'ADN ligase T4.

Les ligatures sont réalisées dans un volume final de 20 *µ*l , à une température de 7 à 12 °C pendant toute la nuit à l'aide de l'ADN ligase du bactériophage T₄ (BRL). La composition du tampon de ligation est la suivante :
- Tris HCl 50 mM pH 7,6
- MgCl₂ 10 mM
- ATP 1 mM
- DTT 1 mM
- PEG-8000 5 % (p/v)

Les quantités relatives de vecteur et de fragment à insérer sont calculées de façon à avoir un excès de fragment à cloner d'un facteur 2. L'efficacité de la ligature peut être contrôlée par électrophorèse en gel d'agarose .

### 10. Transfert d'ADN sur membrane de nitrocellulose.

Après électrophorèse sur gel d'agarose, l'ADN est transféré sur une membrane de nitrocellulose ( Hybond-C extra, Amersham) selon la technique décrite par SOUTHERN ( J. Mol. Biol. 98, 503-517, 1975 ).

Après migration, le gel est photographié sous lumière UV ( 254 nm) puis baigné successivement 1 heure dans un bain de dénaturation (NaOH 0,5 M, NaCl 1,5 M) puis une heure dans un bain de neutralisation (Tris 0,5 M, NaCl 1,5 M).

Le transfert s'effectue durant la nuit dans du 10 X SSC préparé à partir d'une solution stock 20X SSC (NaCl 3 M, tricitrate de sodium 0,3 M pH 7).

L'ADN qui a été transféré est fixé définitivement sur la membrane par séchage sous vide à 80°C pendant 2 heures.

### 11. Marquage radioactif d'une sonde moléculaire par extension d'amorce au hasard ( " random priming" ).

### 11. 1- Principe

Cette méthode a été développée par FEINBERG et VOGELSTEIN ( Anal. Biochem. 132, 6, 1983; Anal. Biochem. 137, 266, 1984) . Le marquage du fragment choisi comme sonde est réalisé par amorçage au hasard suivi d'une extension à l'aide du fragment de Klenow de l'ADN polymérase d'E.coli.

L'ADN dénaturé est incubé dans un milieu contenant un mélange d'hexanucléotides, les quatre désoxynucléotides froids et du [gamma ⁻³² P ] dCTP. L'action du fragment de Klenow de l'ADN polymérase I conduit à la synthèse d'une sonde radioactive dont l'activité spécifique se situe entre 2 et 5 10⁸ cpm par *µ*g d'ADN.

### 11.2 - Aspects pratiques.

L'ADN à marquer ( 25 à 100 ng) est dénaturé par la chaleur (100°C pendant 5 min ), le tube est ensuite placé à 4°C . Le milieu réactionnel suivant est ajouté:
- Mg Cl₂ 1,2 mM
- β-mercaptoéthanol 2,5 mM
- Tris-HCl pH 8 1,2 mM
- Hepes pH 6,6 50 mM
- d NTP 10 mM
- [ gamma-³²P] d CTP 10 mCi.ml⁻¹ 50 *µ*Ci
- Hexamères 0,3 UDO₂₆₀ nm
- Fragment de Klenow 4 U
- BSA 0,4 mg.ml⁻¹
- Eau distillée q.s.p. 50 *µ*l

L'ensemble est incubé pendant environ 1 heure à 37°C. La réaction est arrêtée par addition d'un dixième de volume de d'EDTA 0,5 M.

### 12. Hybridation moléculaire et autoradiographie

La membrane de nitrocellulose portant l'ADN transféré et fixé est soumise à une étape de préhybridation à 68°C pour une durée de 1 à 2 heures, dans environ 10 ml de la solution suivante :
- 6X SSC
- 5X Denharts préparé à partir d'une solution 100X:

| | |
|---|---|
| BSA | 2% (p/v) |
| Ficoll | 2% (p/v) |
| PVP | 2% (p/v) |

- SDS 0,5 % (p/v)
- 0,1 mg.ml⁻¹ d'ADN , dénaturé et soumis aux ultra-sons, de sperme de saumon.

On ajoute ensuite à cette solution, la sonde auparavant marquée et dénaturée par la chaleur L'hybridation s'effectue à 68°C pendant une nuit. La préhybridation et l'hybridation sont réalisées dans un sac de polyéthylène hermétiquement soudé.

La membrane est ensuite lavée dans les bains suivants :
- deux fois 15 min dans du 2X SSC à 65°C
- 30 min dans du 2X SSC; 0,1 % SDS à 65°C. Cette dernière étape peut être répétée si nécessaire.

Après séchage, la membrane est enveloppée dans un film plastique étirable, puis placée dans une cassette d'auto-radiographie avec un film Kodak X-ray inséré entre deux écrans intensificateurs. Cette cassette est alors mise à -80°C . La durée de l'exposition peut varier de quelques heures à une semaine selon l'intensité du signal.

### 13. Réaction de polymérisation en chaîne (PCR)

### 13.1- Principe

Le principe de la technique de PCR repose sur l'utilisation d'un couple d'amorces (" primers"), oligonucléotides de synthèse qui délimitent la région à amplifier sur l'ADN matriciel . L'un des deux oligonucléotides est complémentaire d'une partie du brin sens, l'autre oligonucléotide étant complémentaire d'une partie du brin anti sens . Chaque cycle de PCR comprend trois étapes successives :
- dénaturation de l'ADN
- hybridation des amorces sur l'ADN matriciel
- élongation des chaînes complémentaires de l'ADN par la Taq ADN polymérase depuis les extrémités 3'OH.

L'obtention d'une quantité notable d'ADN amplifié nécessite d'effectuer un nombre important de cycles ( entre 25 et 40) . L'efficacité de l'amplification est de l'ordre de 10⁷, ce qui permet d'obtenir au bout de 30 cycles , environ 1*µ*g d'un fragment d'ADN de 300 pb, à partir d'un *µ*g d'ADN génomique .

### 13.2 - Aspects pratiques

### a- Synthèse des oligonucléotides

La synthèse chimique des deux oligonucléotides utilisés est réalisée sur l'appareil Gene Assembler Plus (Pharmacia LKB) . Cet appareil utilise des dérivés de nucléotides de type β-cyanoéthylphosphoramide . Il permet la synthèse de 0,2 *µ* mole d'oligonucléotides avec une efficacité de couplage supérieure à 90% ne nécessitant pas une purification ultérieure des oligonucléotides dans la mesure où ils n'excèdent pas une longueur de 30 bases.

### b - Réaction de PCR

Les réactions de PCR sont réalisées sur l'automate Gene ATAQ Controller ( Pharmacia LKB) . Elles sont effectuées dans des volumes de 50 *µ*l contenant :
- 50 pmoles de chaque amorce
- 1*µ*g d'ADN génomique
- dATP, dCTP, dGTP, dTTP : 2 mM chacun
- tampon de PCR ( Proméga) :

| | |
|---|---|
| Tris-HCl | 10 mM pH 8,4 |
| KCl | 50 mM |
| MgCl₂ | 1,5 mM |
| Gélatine | 0,1 mg.ml⁻¹ |
| Triton X100 | |

- 1,25 U de Taq ADN polymérase ( Proméga)

L'ADN génomique est préalablement dénaturé à 100°C pendant 5 min en présence des amorces et de 50 *µ*l d'huile minérale pour éviter toute évaporation, puis placé immédiatement dans la glace . L'eau , les désoxynucléotides , le tampon de PCR et la Taq ADN polymérase sont déposés sur l'huile . Une brève centrifugation est nécessaire pour permettre à ces derniers composants de traverser l'huile . Après une étape d'élongation à 72°C, 30 cycles de PCR sont effectués, comprenant chacun :
- une étape de dénaturation de 30 sec à 94°C
- une étape d'hybridation de 30 sec à la température choisie
- une étape d'élongation de 2 min à 72°C.

Les produits de PCR sont séparés par électrophorèse sur gel d'agarose.

### II. TECHNIQUES RELATIVES A LA BIOLOGIE VEGETALE

### 1 - Milieux de culture

### 1.1- Milieu de base.

Le milieu de base est dérivé du milieu de MURASHIGE et SKOOG ( Physiol. Plant, 15, 473-497, 1962). A partir de ce milieu , des modifications permettent d'obtenir un certain nombre de milieux aux propriétés différentes suivant l'utilisation souhaitée. Sa composition est indiquée dans le tableau ci-dessous:

| Composition du milieu de base | | | |
|---|---|---|---|
| Macroéléments ( mg/l ) | | Microéléments ( mg/l ) | |
| KNO₃ | 1900 | MnSO₄ 4H₂O | 22,3 |
| NH₄NO₃ | 1650 | ZnSO₄ 7H₂O | 8,6 |
| CaCl₂ 2H₂O | 440 | H₃BO₃ | 6,2 |
| MgSO₄ 7H₂O | 370 | KI | 0,83 |
| KH₂PO₄ | 170 | Na₂MO₄ 2H₂O | 0,25 |
| | | COCl₂ 6H₂O | 0,025 |
| | | CuSO₄ 5H₂O | 0,025 |
| | | FeSO₄ 7H₂O | 27,85 |
| | | Na₂ EDTA | 37,25 |
| | | Vitamines ( mg/l ) | |
| Inositol | 100 | pyridoxine HCl | 0,5 |
| Thiamine HCl | 0,1 | acide nicotinique | 0,5 |
| Saccharose 20 g/l | | | |

### 1.2 - milieu I.

Il s'agit du milieu de base contenant en plus de l'acide naphtalène acétique (ANA) à 0,2 mg/l et de la benzylaminopurine (6-BAP) à 1 mg/l . Il est utilisé pour la mise en culture des disques foliaires, après inoculation par A.tumefasciens.

### 1.3 - milieu II.

Ce milieu correspond au milieu I additionné de céfotaxime à 200 mg/l , antibiotique destiné à bloquer la croissance des agrobactéries, et de kanamycine à 50 mg/l . Il permet le développement et la sélection de colonies cellulaires pouvant générer la formation de bourgeons puis de plantes transformées .

### 1.4 - milieu III

Ce milieu est obtenu par addition de kanamycine (50 mg/l ) au milieu de base . C'est un milieu de repiquage , où l'absence de régulateurs de croissance permet d'induire le développement de racines à partir des plantules régénérées sur milieu II.

### 1.5 - milieu IV.

Il s'agit du milieu II comportant, comme seul antibiotique, de la kanamycine (50 mg.l⁻¹) . Il est utilisé pour les tests de résistance à la kanamycine sur fragments de feuilles .

Tous les milieux décrits ici sont des milieux solides qui contiennent 0,7 % d'agar ( qualité " plant tissue culture ", commercialisé par SIGMA ) . Le pH est ajusté à 5,7 avant stérilisation (115°C pendant 20 min).

### 2. Matériel végétal

### 2-1 - Choix du matériel végétal

Le choix du tabac ( Nicotiana tabacum cv Paraguay Bell) en tant que source d'explants utilisables dans nos expériences de transformation a été guidé par un souci de facilité technique , la notoriété de l'espèce , en matière de culture in vitro, n'étant plus à établir . Il s'agit d'une variété tétraploïde .

### 2-2 - Désinfection des feuilles

Les feuilles destinées aux expériences d'inoculation sont prélevées sur des plants de tabac cultivés en condition non aseptiques . Une désinfection préalable est donc indispensable . Ceci est réalisé en immergeant les feuilles dans une solution d'hypochlorite de calcium à 1 % pendant 20 min. Trois rinçages à l'eau distillée stérile sont ensuite effectués .

### 3 - Technique d'inoculation de disques foliaires.

### 3.1- Choix et principe de la technique.

Le principe de cette technique est simple : des fragments de feuilles sont mis en contact avec les agrobactéries puis transférés sur un milieu induisant la formation de bourgeons et contenant l'agent sélectif. En différents points du bord des fragments, apparaissent des bourgeons dont l'isolement est aisé. Pour obtenir des plantes résultant d'évènements de transformation indépendants , il est nécessaire d'isoler des bourgeons de fragments différents ou de centres de régénération éloignés sur un même fragment . Comparée à la technique de co-culture de protoplastes, cette méthode présente l'avantage de réduire la phase de croissance du tissu indifférencié . Ainsi , on peut réduire l'apparition d'abérrations génétiques induites par la culture in vitro . De plus , grâce à cette technique, la régénération de plantes est plus rapide et les fragments foliaires sont en outre beaucoup moins délicats à manipuler que les protoplastes.

### 3.2 - Aspects pratiques.

Après désinfection des feuilles, la nervure principale, les nervures latérales importantes ainsi que les bords des limbes sont éliminés . Les feuilles sont ensuite découpées en fragments d'environ 1 cm². Les fragments sont déposés , face inférieure vers le haut, à la surface d'une suspension d'agrobactéries obtenue par dilution au dixième d'une culture de la nuit auparavant lavée dans de l'eau distillée stérile . Des témoins de non inoculation sont réalisés en incubant des fragments foliaires dans de l'eau distillée stérile .

Après 15 minutes d'incubation, les fragments sont égouttés sur du papier filtre stérile et transférés , à raison de 5 fragments par boîte de Pétri de 90 cm de diamètre, sur milieu I.

Au bout de 48 heures , les fragments sont prélevés pour être transférés sur milieu II. Des repiquages sur ce même milieu sont ensuite effectués de façon régulière ( toutes les trois semaines ) afin de maintenir l'efficacité de l'agent sélectif .

### 4- Préparation d'échantillons en vue d'observations en microscopie photonique et électronique.

Des fragments d'environ 1 mm sur 2 mm sont prélevés à des niveaux comparables , sur des feuilles de même rang. Ces échantillons sont fixés, post-fixés et déshydratés selon le procédé décrit précédemment (matériel et méthodes , première partie ) . Les inclusions sont réalisées dans un mélange EPON-ARALDITE.

Les coupes semi-fines , destinées aux observations en microscopie photonique, sont colorées au bleu de toluidine. Les coupes ultrafines, destinées aux observations en microscopie électronique, sont contrastées à l'acétate d'uranyle et au citrate de plomb.

### 5. Dosage de la chlorophylle.

La technique de dosage utilisée dérive de la méthode décrite par HOLDEN ( Clorophylls in : Goodwin T.W. -Chemistry and Biochemistry of Plants Pigments ed: Academic Press, 1965, 462-488.)

150 mg de feuilles ( poids frais ) sont broyées, à 4°C, dans un mortier en présence de 1 ml d'acétone à 80%. L'extrait acétonique est recueilli par décantation et conservé au froid , à l'abri de la lumière . Les résidus sont broyés dans 5 ml d'acétone à 80% L'extrait acétonique obtenu à l'issu de ce second broyage est récupéré par décantation. Cette opération est renouvelée le nombre de fois nécessaire pour que l'extrait acétonique, lors du dernier broyage soit incolore . Les solutions acétoniques sont rassemblées et ajoutées au broyat provenant de la dernière extraction. Après une centrifugation de 10 min à 4500 rpm ( rotor Kontron A 8.24) à 4°C, le surnageant est transféré dans une fiole jaugée afin de déterminer le volume de l'extrait acétonique total. Le volume de l'extrait acétonique total est complété à 25 ml.

Le dosage des chlorophylles est basé sur l'absorption de la lumière par les extraits chlorophylliens dans l'acétone à 80% . La concentration en chlorophylle du surnageant acétonique est estimée par mesure de la DO à 652 nm. Elle est ensuite ramenée au poids sec de feuilles et exprimée en mg de chlorophylle par g de feuilles ( poids lyophilisé).

### 6. Détermination des échanges gazeux respiratoires à l'aide de l'oxygraphe.

### 6.1 - Principe.

L'oxygraphe permet la mesure de la concentration en oxygène d'un milieu liquide . L'appareillage comprend :
- une anode d'argent et une cathode de platine reliées par un pont de chlorure de potassium et séparées d'une cellule de mesure par une membrane de TEFLON perméable à l'oxygène
- un boîtier de polarisation de l'électrode de mesure,
- un potentiomètre enregistreur.

Le principe de la méthode consiste à mesurer le courant électrique qui provient de la réaction d'oxydoréduction faisant intervenir l'oxygène dissous , l'électrode polarisée ( ici la cathode de platine) et la réaction d'oxydation d'une anode d'argent . La tension de polarisation est maintenue constante tout au long de l'expérimentation .

### 6.2 - Aspects pratiques .

Pour chaque expérience, l'électrode est calibrée à 0% et à 100% d'oxygène . La valeur de 0% est obtenue par addition, dans le milieu , de cristaux d'hydrosulfite de sodium. La valeur de 100% est obtenue avec de l'eau saturée en oxygène , à la même température que le milieu expérimental.

Les mesures sont réalisées à l'obscurité en milieu agité , sur des fragments de feuilles d'environ 50 mg ( poids frais). Les fragments sont introduits dans la chambre de mesure auparavant remplie d'une solution tampon constituée du mélange carbonate de sodium 0,1 M/bicarbonate de sodium 1 M (1v: 19 v). On enregistre les variations de la concentration en oxygène due à la respiration des tissus dans le milieu réactionnel, pendant un temps donné . La quantité d'oxygène consommée par les tissus , exprimée en *µ* moles d'O₂ par mn et par mg de feuilles ( poids lyophilisé) peut ainsi être déterminée par rapport à la valeur 100% d'oxygène (concentration maximale en oxygène d'une solution saturée d'air,à la température de l'expérience).

### EXEMPLE 1

### Clonage du gène ERG12 dans un vecteur de transfert mobilisable vers A.Tumefasciens.

### 1 - Clonage du gène ERG12 dans le vecteur de transfert.

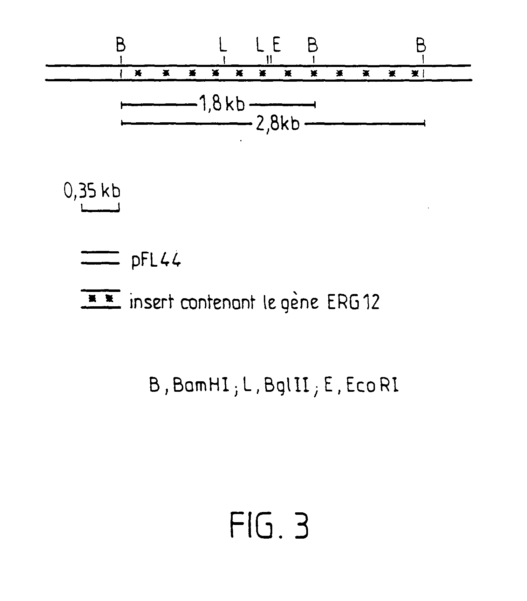

La carte de restriction partielle du fragment Bam H I-Bam H I de 2,8 kb qui contient le gène ERG12 codant pour la MK est représenté figure 3 . Ce fragment de 2,8 kb a été cloné dans le plasmide pFL44 au niveau du site de restriction Bam H I. Le vecteur ainsi obtenu est le pAO11.

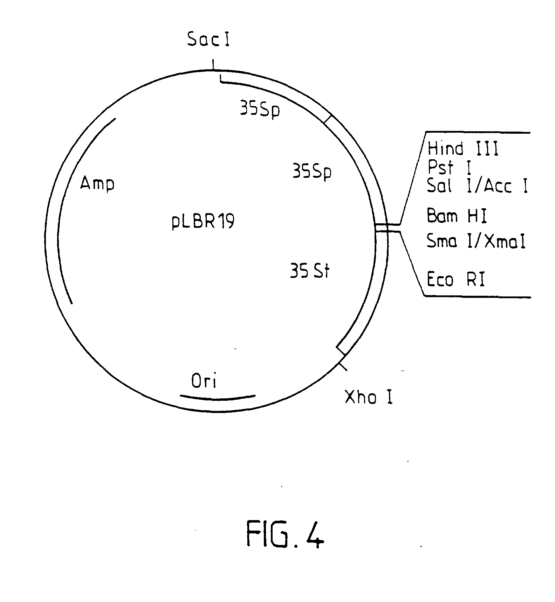

La première étape de la construction a consisté à cloner le gène ERG12 dans le vecteur pLBR 19 représenté sur la figure 4 et qui comprend une cassette d'expression constituée du promoteur ( présent en deux exemplaires disposés en tandem) et de la séquence de polyadénylation de l'ARN 35S du virus de la mosaïque du chou-fleur (CAMV). Un site de clonage multiple a été inséré entre ces deux séquences . Cette région, d'une taille voisine de 1,5 kb, est délimitée par les sites uniques de restriction Sac I et Xho I. Ce vecteur porte en plus une origine de replication fonctionnelle dans E.coli et un marqueur permettant la sélection dans E.coli.

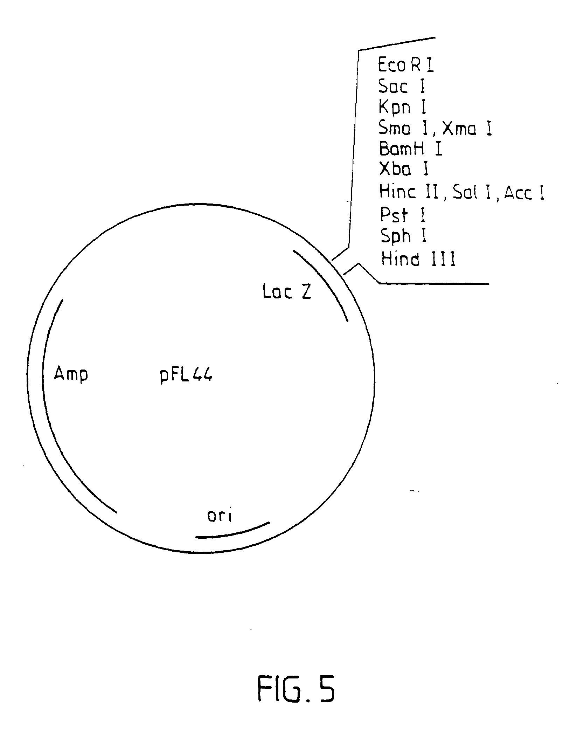

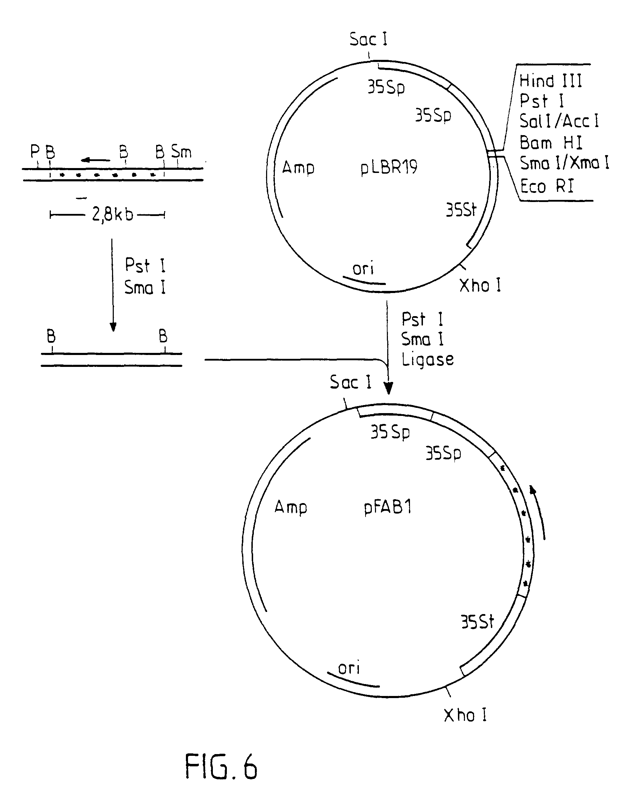

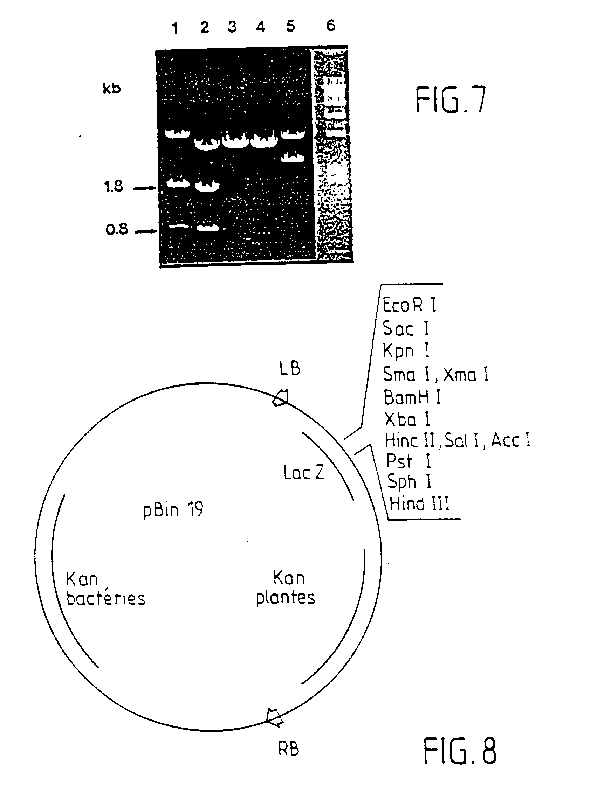

La carte de restriction partielle du plasmide pFL44 ( figure 5), fait apparaître que le fragment Bam H I-BamH I de 2,8 kb contenant le gène ERG12, est bordé par les sites uniques de restriction Pst I et Sma I. Le site multiple de clonage du plasmide pLBR19 représenté sur la figure 4 comporte également ces deux séquences de reconnaissance . Le fragment Pst I-Sma I comprenant l'insert BamH I-BamH I de 2,8 kb, a donc été isolé de pAO11, séparé sur gel d'agarose et cloné, après élution, dans pLBR19 (figure 6). La construction ainsi obtenue (PFAB1 ) a été utilisée pour transformer E. coli 71/18.Les clones contenant le plasmide recombinant ont été sélectionnés sur milieu LB contenant de l'ampicilline. L'ADN plasmidique extrait des colonies sélectionnées a été hydrolysé par BamH I et analysé par électrophorèse sur gel d'agarose à 0,8%. Le profil de restriction obtenu ( figure 7, piste 2) a été comparé à celui du plasmide pAO11 hydrolysé par la même enzyme : deux bandes caractéristiques , l'une correspondant à un fragment de 1,8 kb et l'autre correspondant à un fragment de 0,8 kb , sont observées . Les tailles de ces fragments sont celles attendues en comparaison du profil de restriction provenant de l'hydrolyse de pAO11 par BamH I (figure 7 piste 1).

Afin d'orienter correctement l'insert par rapport au promoteur 35S du CAMV, on a choisi de cloner l'ensemble du fragment [ 35S-ERG12-3' 35S], délimité par les sites de restriction Xho I et Sac I et d'une taille voisine de 4,3 kb,dans le vecteur de transfert Bin19.

La carte de restriction partielle du vecteur de transfert Binl9 est représentée figure 8. L'information génétique que l'on souhaite introduire dans le génome végétal est clonée entre les frontières RB et LB portées par ce plasmide . Ce dernier est introduit dans la souche LBA4404 d'A.tumefasciens hégergeant un plasmide Ti (pTi) dépourvu de la région T. Le transfert de la séquence clonée est alors permis grâce aux fonctions de virulence portées par ce pTi. Le vecteur de transfert Binl9, utilisé dans des systèmes binaires de transformation est compatible avec les plasmides portant les fonctions de virulence et possède :
- une origine de replication fonctionnelle dans E.coli,
- une origine de replication fonctionnelle dans A.tumefasciens,
- un marqueur de sélection bactérien (gène codant pour la résistance à la kanamycine),
- un gène chimérique de résistance à la kanamycine permettant la sélection des cellules végétales transformées . Ce gène est inséré entre les séquences bordures droite et gauche du plasmide et est co-transféré dans le génome des cellules végétales.

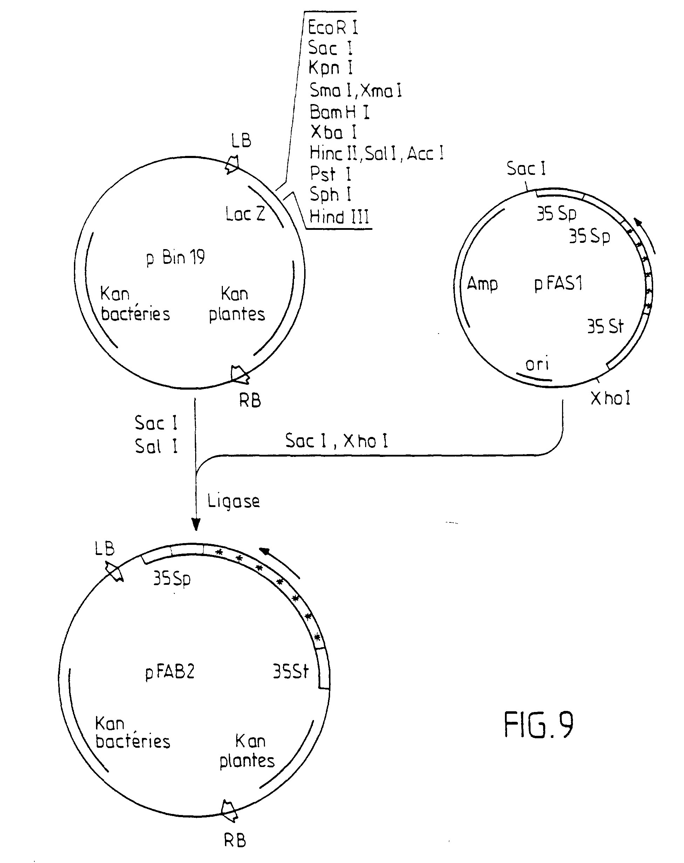

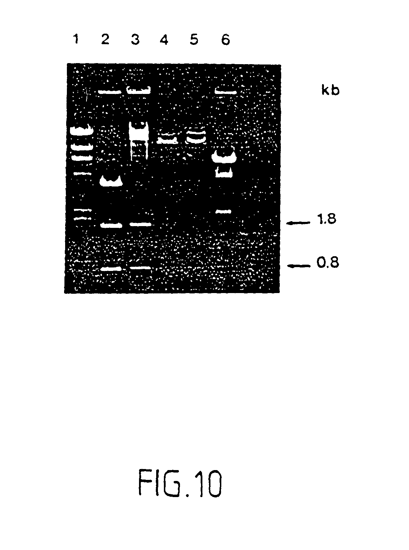

Le fragment XhoI- Sac I comportant le gène ERG12 a été isolé du vecteur pFAB1 et séparé sur gel d'agarose . Après élution, il a été cloné dans le vecteur de transfert au niveau des sites Sac I et Sal I qui génèrent des extrémités compatibles avec celles provenant d'une hydrolyse par Xho (figure 9 ) . La construction résultant de ce clonage (pFAB2) a été introduite, par transformation dans E.coli HB101 et les clones contenant le plasmide recombinant ont été sélectionnés sur milieu LB contenant de la kanamycine . Comme précédemment, l'ADN plasmidique extrait des colonies sélectionnées a été hydrolysé par BamH I et analysé par électrophorèse sur gel d'agarose à 0,8 %. Les bandes caractéristiques ( 1,8 kb et 0,8 kb) ont été retrouvées (figure 10, piste 3 ).

### 2. Introduction du vecteur de transfert pFAB2 portant le gène ERG12 dans A.tumefasciens.

Le vecteur pFAB2 a été introduit par conjugaison dans la souche LBA4404 d'A.tumefasciens, résistante à la streptomycine et à la rifampicine en présence d'une souche "helper" d'E.coli comportant le plasmide pRK2O13 porteur d'un gène de résistance à la kanamycine . Ce plasmide, incapable de se repliquer dans A. tumefasciens est perdu , à l'inverse du vecteur de transfert pFAB2 qui lui, est conservé et peut être amplifié. Seuls les clones d'A.tumefasciens qui ont reçu ce vecteur seront capables de se développer sur un milieu AP contenant de la kanamycine, de la streptomycine et de la rifampicine.

Lors des expériences de conjugaison, des colonies résistantes à ces trois antibiotiques ont été obtenues aussi bien en présence qu'en absence du plasmide "helper" . Les colonies ainsi sélectionnées ont été cultivées en milieu AP liquide contenant de la kanamycine et celles qui ont été capables de croître sur ce milieu ont été retenues pour les expériences d'inoculation de disques foliaires.

### 3 - Essais qualitatifs d'activité MK

Ce test , mis au point par SERVOUSE et KARST (Biochem. J., 240; 541-547 , 1986) permet de contrôler de façon simple et rapide la présence ou l'absence d'une activité MK dans les bactéries transformées par les différents plasmides recombinants.

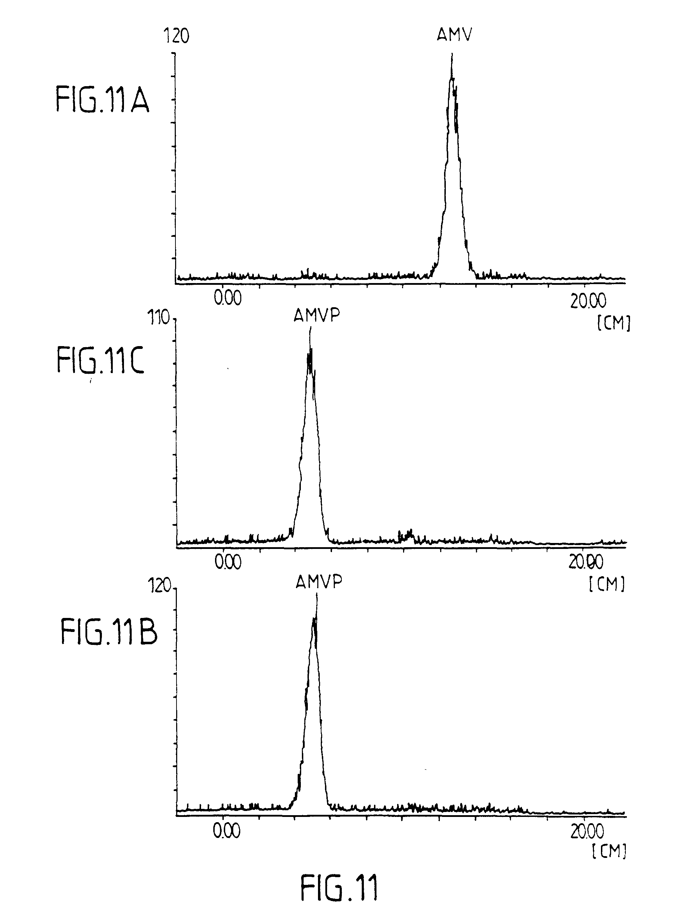

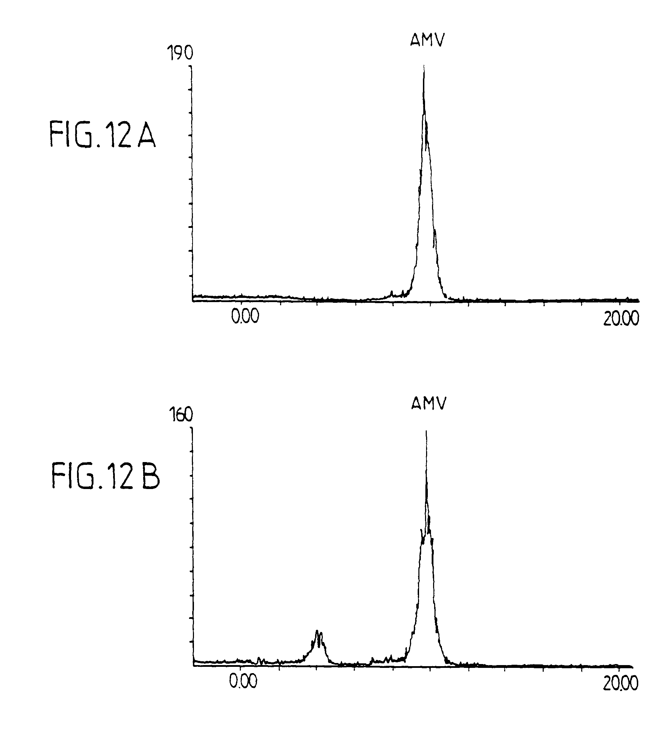

Le contenu d'une petite anse de bactéries préincubées la veille sur milieu solide approprié , est mis en suspension dans du tampon phosphate 0,1 M pH 7,4. Une goutte de toluène est ajoutée afin de perméabiliser les cellules . Après une incubation à 37°C pendant 30 min, les cellules sont mises en contact avec le mélange réactionnel (ATP 20 mM, MgCl₂ 10 mM,[¹⁴ C ] AMV 1,5 mM) et incubées 15 à 30 min à 35°C. La réaction est bloquée par chauffage à 100°C pendant 5 min. Après centrifugation, le surnageant est analysé par chromatographie sur couche mince de gel de silice dans le système n-propanol-ammoniac-eau (6v: 3v: 1v). La distribution de la radioactivité, déterminée par lecture de la plaque avec un scanner de radioactivité Berthold 2832 automatic TLC linear Analyser, permet de visualiser la transformation de l'AMV en AMVP (figures 11 et 12).

Ce test, effectué pour la souche d'E.coli porteuse du vecteur pFAB1 ( figure 11C) ainsi que pour la souche d'A.tumefasciens porteuse du vecteur pFAB2, a permis de déceler dans chaque cas , une activité MK . Les souches E.coli 71/18 [pFL44] ( figure 11A) et E.coli Hb 101 [ pBin 19] (figure 12A) constituent les témoins négatifs . La souche E.coli 71/18 [ pAO11] (figure 11B) constitue , quant à elle , le témoin positif.

### EXEMPLE 2:

### Transfert du gène et vérification de sa présence dans le génome des plants de tabac régénérés.

### 1- Obtention des plantes transformées.

Des fragments de feuilles de plants de tabac ont été traités par la souche d'A.tumefasciens porteuse du vecteur pFAB2 selon la technique d'inoculation de disques foliaires décrite dans le chapitre "matériel et méthodes ". Les fragments ont été placés sur milieu de régénération contenant de la kanamycine (milieu I). Des fragments témoins de résistance à la kanamycine (notés " témoins Kan^{R}") ont été mis en culture sur ce même milieu après inoculation par la souche d'A.tumefasciens porteuse du plasmide Bin19. Enfin, des fragments témoins de régénération ( notés " témoins H₂O") ont été cultivés sur milieu de régénération sans kanamycine après avoir été incubés dans de l'eau.

Une cinquantaine de plantes résultant d'évènements de transformation indépendants ont été transférées en serre. Elles proviennent de quatre expériences d'inoculation différentes . Ces plantes sont fertiles puisqu'elles ont fleuri et donné des graines.

### 2- Test de résistance à la kanamycine.

Ce test consiste simplement à induire, sur milieu contenant de la kanamycine (milieu IV), la formation de cals à partir d'un fragment foliaire . Il est rapide et sans ambiguité .

Des feuilles prélevées sur les plantes issues d'expériences de transformation et de plantes " témoins H₂O " sont découpées en fragments d'environ 1 cm² . Les fragments sont ensuités placés sur milieu IV.

Les fragments qui proviennent de plantes "témoins H₂O" blanchissent très rapidement lorsqu'ils sont cultivés en présence de kanamycine contrairement aux fragments issus de plantes transformées, à partir desquelles, il est de nouveau possible d'induire une régénération.

### 3. Contrôle de la présence du gène .

### 3.1 - Southern blot

L'ADN génomique des plantes transformées a été isolé selon la technique de ROGERS et BENDICH (Plant Mol. Biol.5 , 69-76 1985), décrite dans " matériel et méthodes " . Il a été hydrolysé par Bam HI, puis après électrophorèse, transféré sur membrane de nitrocellulose. Il a été ensuite hybridé avec une sonde correspondant au fragment Bam HI-Bam HI de 1,8 kb qui contient probablement la plus grande partie du gène ERG12 (OULMOUDEN et KARST, Curr. Genet,19, 1991,9-14, 1990 ).

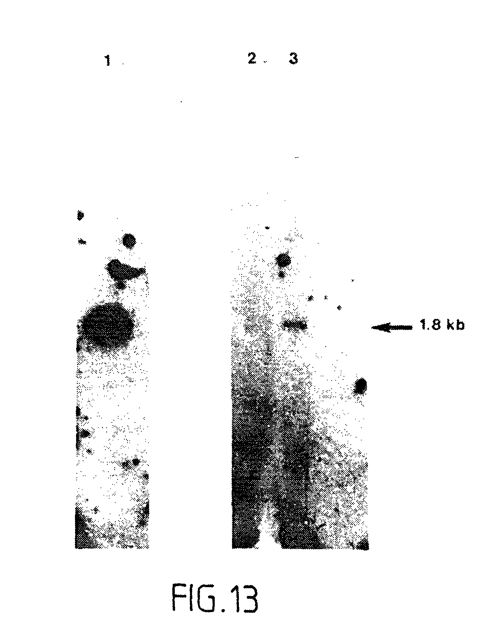

La figure 13 représente les résultats d'une expérience d'hybridation: on retrouve, au niveau de la piste correspondant à l'ADN extrait d'une plante transformée ( piste 3), un fragment dont la taille est celle que l'on pouvait attendre (1,8 kb), montrant ainsi que l'intégration du gène ERG12 a bien eu lieu. Cette série d'hybridation comporte également deux témoins:
- un témoin correspondant à l'hydrolyse par Bam HI de l'ADN génomique d'une plante , " témoin H₂O", hybridé avec la même sonde (piste 2) : aucune bande n'est observée,
- un témoin correspondant à l'hydrolyse par Bam HI de l'ADN du plasmide pAO11 (vecteur d'où a été isolé le gène ERG12 et qui constitue le témoin positif), hybridé avec la même sonde : on retrouve une bande correspondant à un fragment d'une taille de 1,8 kb (piste 1).

Il convient cependant de noter que l'obtention d'une réponse claire et sans ambiguité , par cette technique , nécessite , en particulier chez les végétaux , l'extraction d'une quantité relativement importante d'ADN. Ceci est à mettre en relation avec la taille importante des génomes végétaux et avec le faible nombre de copies généralement présentes dans le génome des plantes transformées par les agrobactéries .

### 3-2 - Réaction de polymérisation en chaîne (PCR).

### a- choix des amorces

Les deux oligonucléotides (appelés ON₁ et ON₂) synthétisés chimiquement et utilisés comme amorce dans les réactions de PCR ont été choisis de façon à avoir à l'intérieur du fragment amplifié , un site unique reconnu par l'enzyme de restriction Bam HI. Leurs séquences sont les suivantes :
- ON₁ 5 ATGTCATTACCGTTCTTAACTTCT 3'
- ON₂

5'GCTTGAGCCCAACCC 3'

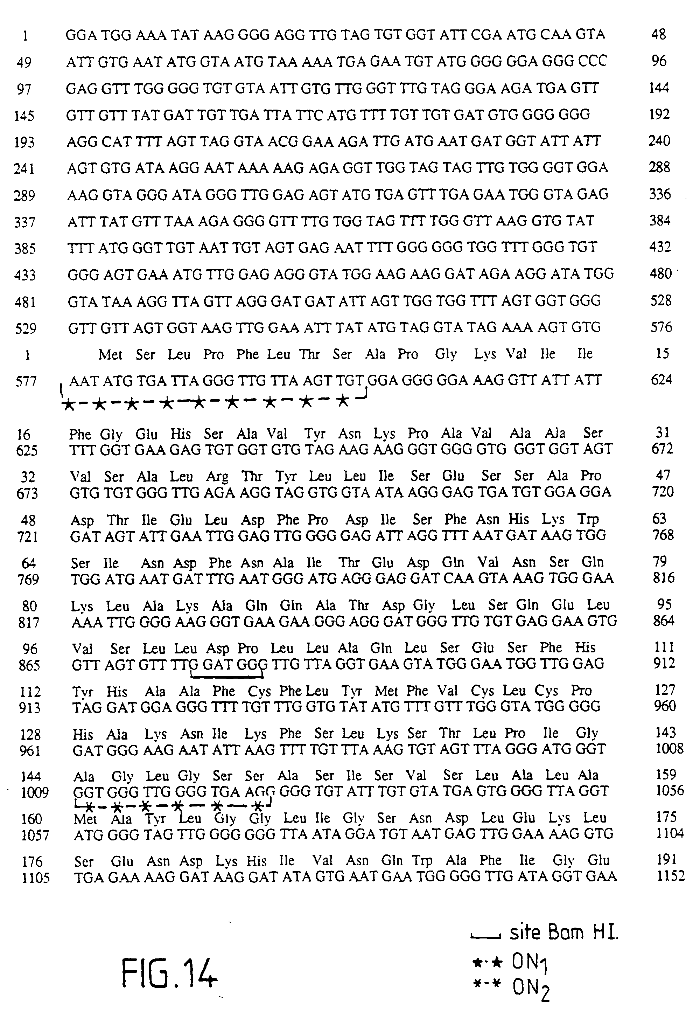

La localisation de ces deux oligonucléotides et celle du site Bam HI, au niveau de la séquence nucléotidique du gène ERG12 est représentée figure 14. La partie amplifiée in vitro doit correspondre à un fragment de 447 pb. Une hydrolyse par Bam HI du fragment amplifié doit libérer deux fragments de 150 et 297 pb.

### b - résultat de l'amplification.

Le choix d'une température d'hybridation de 45°C a été guidé par la température de fusion des amorces (Tm) qui détermine l'hybridation de ces mêmes amorces sur l'ADN à amplifier.

Une bande d'une taille voisine de 447 pb est amplifiée avec le couple d'amorces ON₁ et ON₂ pour les échantillons correspondant à l'ADN génomique des plantes transformées ( figure 15, pistes 1 à 6) de même que pour l'échantillon d'ADN génomique de levure qui constitue le témoin positif ( figure 15, piste 7). Les résultats qui concernent les différentes plantes transformées ont été obtenus après suramplification. Cette étape permet de mieux visualiser les bandes dont l'intensité était faible , dans certains cas, après la première amplification .

Aucune bande d'amplification n'est obtenue avec les amorces ON₁ et ON₂ seules, à partir de l'ADN génomique de levure ( figure 15, pistes 8 et 9): la bande d'amplification visible au niveau des pistes 1 à 7 résulte donc bien d'une amplification obtenue avec les deux amorces.

Aucune bande d'amplification n'est observée dans le cas d'ADN génomique isolé de plante " témoin H₂O" (figure 15, piste 10).

Un autre contrôle consistant à remplacer l'ADN par de l'eau stérile prouve qu'aucun des constituants utilisés n'était contaminé par de l'ADN exogène puisqu'aucune bande d'amplification n'est observée dans ce cas ( figure 15, piste 11).

### c- analyse du fragment amplifié.

L'obtention d'une bande d'une taille voisine de 447 pb ne suffit pas pour affirmer qu'il s'agit bien d'une amplification à partir du gène ERG12. Le fragment amplifié a donc été élué après migration, hydrolysé par Bam HI et analysé par électrophorèse sur gel d'agarose. Deux bandes sont observées; l'une correspond à un fragment d'une taille voisine de 297 pb et l'autre à un fragment d'une taille voisine de 150 pb. Ce profil est obtenu aussi bien à partir d'ADN génomique de levure (figure 16, piste 2) qu'à partir d'ADN génomique de plantes transformées (figure 16, piste 1). Le fragment amplifié, dans ce dernier cas, correspond donc bien au gène ERG12.

### 3.3- Conclusion.

L'ensemble des résultats obtenus par Southern blot et par PCR montre donc que l'intégration du gène ERG12 dans le génome des plantes transformées a bien eu lieu.

### EXEMPLE 3 :

### Etude des effets de l'insertion.

### 1) Effets sur la croissance et sur la régénération des plantes transformées.

### 1.1 Régénération

### a- observations.

Deux semaines sont nécessaires pour voir apparaître,chez les "témoins H₂O", un début de callogenèse qui prend place tout d'abord sur le bord des fragments mis en culture et qui envahit ensuite progressivement la surface des explants ; au bout d'un mois , le cal est assez abondamment développé . A ce stade , la différenciation de racines précède très souvent l'organisation de bourgeons.

Chez les " témoins Kan^{R}" , la callogenèse est faible.Il y a formation de bourgeons entre trois semaines et un mois après la mise en culture des explants. Cette différenciation semble pouvoir s'initier à partir de cals peu développés mais également de manière plus directe, en différents points du pourtour du fragment.

Pour les fragments traités par la souche d'A.tumesfasciens porteuse du vecteur pFAB2 , la différenciation intervient sans que l'on puisse auparavant discerner la formation d'un cal. Des bourgeons sont visibles, en différents points du bord des explants, trois semaines après leur mise en culture. Ceci est comparable à ce que l'on observe pour les fragments " témoins Kan^{R}" . Cependant, le développement ultérieur de ces bourgeons semble beaucoup plus rapide . Simultanément, une callogenèse qui semblait pratiquement inexistante au départ, prend place . A partir de ces groupes de cellules indifférenciées, peuvent se différencier de nouveaux centres organogènes qui donneront naissance à de nouveaux bourgeons. Ainsi, le nombre de centres de régénération, au niveau des fragments traités par la souche d'A.tumefasciens porteuse du vecteur pFAB2 est plus élevé que pour les "témoins H₂O" et "Kan^{R}". Il en résulte un nombre également plus important de plantules régénérées .

Les plantules en cours de régénération sont excisées et repiquées en boîte de Pétri sur milieu sans hormone mais contenant toujours l'agent sélectif (milieu III), afin de favoriser le développement de racines . Les plantules qui proviennent des fragments "témoins H₂O" sont, quant à elles, transférées sur milieu sans hormone et sans agent sélectif . Lorsque les racines apparaissent, les plantes sont repiquées sur le même milieu contenu dans des boîtes de culture Magenta GA₃ (Poly Labo) pour permettre leur développement . Quelques semaines après cette étape (en général deux semaines), une différence de croissance notable peut déjà être observée :les feuilles des plantes régénérées à partir d'explants inoculés par la souche d'A.tumefasciens porteuse du vecteur pFAB2 sont plus développées, les entrenoeuds sont plus courts et les tiges semblent beaucoup plus "trapues", ceci en comparaison des plantes " témoins H₂O" et "Kan^{R}" . Les plantes " témoins Kan^{R}" ont une allure similaire aux plantes " témoins H₂O" . Deux mois après le transfert en boîte de culture Magenta, les plantes régénérées sont transférées en condition non aseptique .

Il faut donc environ quatre mois, à compter du jour de l'inoculation, pour obtenir des plantes complètes qui soient capables de croître après transfert en terre. Ces plantes sont ensuite maintenues sous cloche durant un mois au laboratoire sous photopériode naturelle, afin de réduire au maximum les traumatismes provoqués par le sevrage. Elles sont, après cette période d'acclimatation, cultivées en serre.

### b - Conclusion.

Comparés aux différents témoins, les processus intervenant dans la régénération des plantes ayant intégré dans leur génome le gène ERG12, se caractérisent par :
- une réduction plus ou moins importante de la phase cal au profit d'une organogenèse plus précoce se traduisant par une différenciation presque directe de bourgeons,
- un nombre de centres de régénération plus élevé consécutif à une différenciation s'initiant à partir de centres secondaires,
- un développement des bourgeons beaucoup plus rapide,
- des plantes d'aspect plus "trapu" avec des feuilles plus développées.

### 1.2 - Caractéristiques morphologiques et croissance des plantes transformées .

### a. Observations.

Un suivi régulier des plantes transformées , cultivées en serre, a été effectué . Leur croissance et leurs caractéristiques morphologiques ont été comparées uniquement à celle des plantes " témoin H₂O" .

Le phénomène de précocité observé lors de la régénération, au niveau de la différenciation des bourgeons, se maintient chez les plantes adultes Cette précocité, très marquée durant la phase de croissance des plantes , s'atténue toutefois légèrement à mesure que l'on s'achemine vers la floraison. Dans la majeure partie des cas, la floraison des plantes ayant intégré dans leur génome le gène ERG12 et celle des plantes témoins sont décalées dans le temps ( de deux à trois semaines ). Ce décalage est toujours favorable aux plantes transformées . Pour un cas particulier , un décalage allant dans le même sens mais , cette fois-ci, beaucoup plus marqué ( de l'ordre de trois mois ) , a été observé sans que l'on puisse savoir si cela était une conséquence extrême de l'insertion du gène .

Le phénomène d'accélération de la croissance, observé initialement , lors du développement des bourgeons , se maintient également chez les plantes adultes. Les plantes transformées ont une taille plus importante et un nombre de rangs plus élevé ( accélération du plastochrone). Leurs entrenoeuds sont maintenant plus développés que chez les plantes témoins. Ce phénomène s'atténue à mesure que l'on se rapproche de la floraison. La surface foliaire est augmentée (facteur variant de 1,3 à 1,5), en particulier pour les feuilles situées à la base de la plante.

Enfin, les plantes transformées montrent des inflorescences plus ramifiées. Le nombre de fleurs par inflorescence est augmenté ( facteur 3 pour les inflorescences principales ). Le nombre de graines récoltées est donc plus élevé.

### b - Conclusion.

La croissance des plantes ayant intégré dans leur génome le gène ERG12, est, tout comme leur régénération, marquée par un caractère de précocité et de rapidité qui semble cependant légèrement s'atténuer à l'approche de la floraison et qui conduit à un important développement des inflorescences.

### 2. Analyses histologique et infrastructurale

Cette étude , réalisée en parallèle sur des feuilles de plantes transformées et de plantes " témoin H₂O" , a été entreprise dans le but de rechercher si les différences morphologiques observées lors de la croissance des plantes régénérées pouvaient être reliées à des modifications de nature histologique et infrastructurale . Des coupes transversales ont été obtenues à partir de fragments provenant de plantes agées de 6 mois.

Les observations histologiques, accompagnées de mesures sur clichés photographiques , permettent de mettre en évidence, chez les plantes transformées , une diminution de la taille moyenne des cellules du mésophylle, par rapport aux plantes " témoin H₂O". Cette diminution, de l'ordre de 20 à 30% dans tous les cas examinés, implique pour un même volume, une augmentation du nombre de cellules d'une valeur équivalente. Etant donné que les organes, en particulier les feuilles, ont une taille moyenne augmentée d'un facteur 1,5 et que les entrenoeuds ont également une longueur accrue, on peut conclure à une intense prolifération cellulaire dans l'ensemble de la plante . On constate par exemple que le parenchyme palissadique apparaît bisérié alors qu'il est unisérié dans les feuilles des témoins.

L'analyse infrastructurale des cellules du mésophylle fait également apparaître un certain nombre de modifications, qui concerne en particulier les mitochondries et les chloroplastes.Chez les " témoins H₂O" , les plastes des cellules du parenchyme palissadique sont du type amylochloroplastes ,caractérisés par la présence de nombreuses et volumineuses lentilles d'amidon intrastromatiques . Ces lentilles sont nettement moins nombreuses et de taille beaucoup plus réduite dans les cellules des plantes transformées . On note également que chez ces dernières, les mitochondries sont nombreuses, de petite taille, de forme sphérique tandis que chez les témoins, elles paraissent plus allongées et moins nombreuses . De plus , la densité en ribosomes cytosoliques apparaît plus importante chez les plantes transformées . Tous ces caractères indiquent une activité métabolique plus intense chez les plantes transformées .

### 3- Dosage de la chlorophylle.

Cette analyse a été entreprise dans le but de déterminer si la couleur verte des feuilles plus intense , observée au niveau des plantes transformées par rapport à celle des plantes témoins , pouvait être le reflet de modifications de la teneur en chlorophylle totale (chlorophylle a et chlorophylle b).

Plusieurs séries de dosages, réalisés sur des feuilles de niveau moyen provenant de plantes transformées et de plantes " témoin H₂O" permettent de mettre en évidence une augmentation du taux de chlorophylle totale ( valeurs ramenées au poids de matériel lyophilisé) d'un facteur variant de 2,5 à 3 chez les plantes transformées . Il faut toutefois noter que, lorsque l'on s'adresse à des feuilles de la partie apicale des plantes, cette différence n'est pas retrouvée .

### 4 - Mesure des échanges gazeux respiratoires.

Ces mesures , réalisées à l'aide de l'oxygraphe, à partir de fragments de feuilles situées à la base de plantes agées de six mois, ont été effectuées en parallèle chez les plantes transformées et chez les plantes témoins. Elles ont permis de mettre en évidence une modification des échanges gazeux respiratoires . En effet, chez les plantes transformées, ces échanges sont augmentés d'un facteur variant de 1,6 à 2 par rapport aux plantes " témoin H₂O " et ceci pour une même surface foliaire . Ces résultats peuvent être reliés à ceux apportés par les observations ultrastructurales concernant notamment les mitochondries et indiquent une activité métabolique plus intense chez les plantes transformées .

### 5. Transmission héréditaire du gène .

Les plantes transformées ont donné des graines qui majoritairement permettent l'obtention de plantes résistantes à la kanamycine .

Seulement un petit nombre (inférieur à 5% ) de plantes issues de ces graines redevient sensible

## Revendications

1. Plante **caractérisée en ce qu'**elle porte dans son génome un ou plusieurs gènes exprimant une ou plusieurs enzymes des étapes précoces de la voie de biosynthèse des phytostérols constituées par la chaîne de réactions biochimiques par laquelle l'acide mévalonique est transformé en farnésyl diphosphate, lesdits gènes soit ne se retrouvant pas dans la plante native, soit étant originaires de ladite plante, auquel cas ils sont présents dans un nombre de copies supérieur à celui de la plante native ou ils sont modifiés dans leur structure ou leur fonction par rapport à ceux de la plante native.

2. Plante selon la revendication 1, **caractérisée en ce que** ladite étape précoce est la transformation de l'acide mévalonique en mévalonyl 5-phosphate .

3. Plante selon l'une des revendications 1 et 2, **caractérisée en ce qu'**elle porte un ou plusieurs gènes codant pour une ou plusieurs mévalonate-kinases.

4. Plante selon la revendication 3, **caractérisée en ce que** la mévalonate kinase est codée par le gène ERG12, l'un de ses dérivés ou fragments exprimant une activité mévalonate kinase.

5. Plante selon l'une quelconque des revendications 1 à 3 **caractérisée en ce qu'**elle porte un ou plusieurs gènes codant pour l'activité mévalonate kinase chez les crucifères , et en particulier chez Arabidopsis ou chez le colza.

6. Plante selon la revendication 1, **caractérisée en ce qu'**elle porte un ou plusieurs gènes codant pour une farnésyl diphosphate synthétase.

7. Plante selon la revendication 6, **caractérisée en ce que** la farnésyl diphosphate synthétase est codée par le gène ERG20 de la levure , ou l'un de ses dérivés ou fragments exprimant une activité farnésyl diphosphate synthétase.

8. Plante selon la revendication 1, **caractérisée en ce qu'**elle porte un ou plusieurs gènes codant pour une AMVP kinase.

9. Plante selon la revendication 8, **caractérisée en ce que** l'AMVP kinase est codée par le gène ERG8, ou l'un de ses dérivés ou fragments exprimant une activité AMVP kinase.

10. Plante selon l'une des revendications 1 à 9, **caractérisée en ce qu'**elle appartient à la catégorie des plantes oléoprotéagineuses .

11. Plante selon l'une des revendications 1 à 10, **caractérisée en ce qu'**elle appartient à la famille des crucifères et en particulier fait partie des espèces colza.

12. Plante selon l'une des revendications 1 à 10, **caractérisée en ce qu'**elle fait partie de l'espèce tournesol.

13. Plante selon l'une des revendications 1 à 9, **caractérisée en ce qu'**elle fait partie des espèces Vigna radiata ou aubergine.

14. Plante selon l'une des revendications 1 à 10, **caractérisée en ce qu'**elle appartient à la famille des Fabacées et en particulier fait partie de l'espèce soja.

15. Plante selon l'une des revendications 1 à 14, **caractérisée en ce que** le ou les gènes sont placés sous le contrôle d'un promoteur inséré à proximité du ou des gènes d'une manière à permettre l'expression de leurs produits biologiquement actifs .

16. Vecteur pour l'insertion d'au moins un gène codant pour une ou plusieurs enzymes des étapes précoces de la voie de biosynthèse des phytostérols constituées par la chaine de réactions biochimiques par laquelle l'acide mévalonique est transformée en farnésyl diphosphate, dans des cellules végétales, **caractérisé en ce qu'**il comprend :
- une séquence du gène à insérer,
- une ou plusieurs séquences promoteur en amont du gène à insérer ,
- une ou plusieurs séquences terminateur en aval du gène à insérer ,
- un ou plusieurs marqueurs permettant la sélection des cellules dans lesquelles le gène s'est inséré , et
- des séquences d'un plasmide Ti permettant le transfert desdits gènes et marqueurs d'une bactérie vers une cellule végétale , lesdits gènes et marqueurs étant orientés et insérés , de manière à permettre leur expression.

17. Vecteur selon la revendication 16,**caracterisé en ce que** le gène code pour une mévalonate kinase

18. Vecteur selon la revendication 17, **caractérisé en ce que** le gène est le gène ERG12.

19. Plasmide pFAB2 selon la revendication 18 , **caractérisé en ce qu'**il a été déposé sous le n° I-1176 du 14 Février 1992 auprès de la Collection Nationale de Culture des Microorganismes de l'Institut Pasteur (CNCM).

20. Procédé de traitement d'une plante par insertion dans son génome d'un gène codant pour une ou plusieurs enzymes des étapes précoces de la voie de synthèse des phytostérols constituées par la chaine de réactions biochimiques par laquelle l'acide mévalonique est transformée en farnésyl diphosphate.

21. Procédé selon la revendication 20 , **caractérisé en ce que** l'insertion est effectuée par transfert dans des cellules individualisées ou dans des groupes de cellules d'un vecteur selon l'une des revendications 16 à 19 , éventuellement en présence d'un autre vecteur de type plasmide Ti ayant des fonctions permettant ce transfert .

## Patentansprüche

1. Pflanze, **dadurch gekennzeichnet, dass** sie in ihrem Genom ein oder mehrere Gene trägt, die ein oder mehrere Enzyme der frühen Schritte des Biosynthesewegs der Phytosterole exprimieren, welche aus der Kette biochemischer Reaktionen bestehen, durch die Mevalonsäure in Farnesyldiphosphat umgewandelt wird, wobei die Gene entweder in der nativen Pflanze nicht vorkommen oder aus der Pflanze stammen, in welchem Fall sie in größerer Kopienzahl vorhanden sind als in der nativen Pflanze oder in ihrer Struktur oder Funktion in Bezug auf die native Pflanze modifiziert sind.

2. Pflanze gemäß Anspruch 1, **dadurch gekennzeichnet, dass** es sich bei dem frühen Schritt um die Umwandlung von Mevalonsäure in Mevalonyl-5-phosphat handelt.

3. Pflanze gemäß einem der Ansprüche 1 und 2, **dadurch gekennzeichnet, dass** sie ein oder mehrere Gene trägt, die für eine oder mehrere Mevalonat-Kinasen codieren.

4. Pflanze gemäß Anspruch 3, **dadurch gekennzeichnet, dass** die Mevalonat-Kinase durch das Gen ERG12 oder eines seiner Derivate oder Fragmente, die eine Mevaionat-Kinase-Aktivität exprimieren, codiert ist.

5. Pflanze gemäß einem der Ansprüche 1 bis 3, **dadurch gekennzeichnet, dass** sie ein oder mehrere Gene trägt, die bei den Kreuzblütlern und insbesondere bei *Arabidopsis* oder bei Raps für die Mevalonat-Kinase-Aktivität codieren.

6. Pflanze gemäß Anspruch 1, **dadurch gekennzeichnet, dass** sie ein oder mehrere Gene trägt, die für eine Farnesyldiphosphat-Synthetase codieren.

7. Pflanze gemäß Anspruch 6, **dadurch gekennzeichnet, dass** die Farnesyldiphosphat-Synthetase durch das Gen ERG20 der Hefe oder eines seiner Derivate oder Fragmente, die eine Farnesyldiphosphat-Synthetase-Aktivität exprimieren, codiert ist.

8. Pflanze gemäß Anspruch 1, **dadurch gekennzeichnet, dass** sie ein oder mehrere Gene trägt, die für eine AMVP-Kinase codieren.

9. Pflanze gemäß Anspruch 8, **dadurch gekennzeichnet, dass** die AMVP-Kinase durch das Gen ERG8 oder eines seiner Derivate oder Fragmente, die eine AMVP-Kinase-Aktivität exprimieren, codiert ist.

10. Pflanze gemäß einem der Ansprüche 1 bis 9, **dadurch gekennzeichnet, dass** sie zur Kategorie der öl- und proteinhaltigen Pflanzen gehört.

11. Pflanze gemäß einem der Ansprüche 1 bis 10, **dadurch gekennzeichnet, dass** sie zur Familie der Kreuzblütler gehört und es sich insbesondere um eine Rapsart handelt.

12. Pflanze gemäß einem der Ansprüche 1 bis 10, **dadurch gekennzeichnet, dass** es sich um eine Sonnenblumenart handelt.

13. Pflanze gemäß einem der Ansprüche 1 bis 9, **dadurch gekennzeichnet, dass** es sich um eine *Vigna-radiata-* oder Auberginenart handelt.

14. Pflanze gemäß einem der Ansprüche 1 bis 10, **dadurch gekennzeichnet, dass** sie zur Familie der Fabaceen gehört und es sich insbesondere um eine Sojaart handelt.

15. Pflanze gemäß einem der Ansprüche 1 bis 14, **dadurch gekennzeichnet, dass** das oder die Gene unter die Kontrolle eines Promotors gestellt sind, der so in der Nähe des oder der Gene eingesetzt ist, dass er die Expression ihrer biologisch aktiven Produkte ermöglicht.

16. Vektor zum Einschleusen wenigstens eines Gens, das für ein oder mehrere Enzyme der frühen Schritte des Biosynthesewegs der Phytosterole codiert, welche aus der Kette biochemischer Reaktionen bestehen, durch die Mevalonsäure in Farnesyldiphosphat umgewandelt wird, in Pflanzenzellen, **dadurch gekennzeichnet, dass** er folgendes umfasst:
- eine Sequenz des einzuschleusenden Gens;
- eine oder mehrere Promotorsequenzen stromaufwärts des einzuschleusenden Gens;
- eine oder mehrere Terminatorsequenzen stromabwärts des einzuschleusenden Gens;
- ein oder mehrere Marker, die die Selektion der Zellen erlauben, in die das Gen eingeschleust wurde; und
- Sequenzen eines Ti-Plasmids, das die Übertragung der Gene und Marker eines Bakteriums in eine Pflanzenzelle erlaubt, wobei die Gene und Marker so orientiert und eingesetzt sind, dass ihre Expression ermöglicht wird.

17. Vektor gemäß Anspruch 16, **dadurch gekennzeichnet, dass** das Gen für eine Mevalonat-Kinase codiert.

18. Vektor gemäß Anspruch 17, **dadurch gekennzeichnet, dass** es sich bei dem Gen um das Gen ERG12 handelt.

19. Plasmid pFAB2 gemäß Anspruch 18, **dadurch gekennzeichnet, dass** es am 14. Februar 1992 unter der Nr. I-1176 bei der Collection Nationale de Culture des Microorganismes des Institut Pasteur (CNCM) hinterlegt wurde.

20. Verfahren zur Behandlung einer Pflanze zur Einschleusung eines Gens, das für ein oder mehrere Enzyme der frühen Schritte des Biosynthesewegs der Phytosterole codiert, welche aus der Kette biochemischer Reaktionen bestehen, durch die Mevalonsäure in Farnesyldiphosphat umgewandelt wird, in ihr Genom.

21. Verfahren gemäß Anspruch 20, **dadurch gekennzeichnet, dass** die Einschleusung durch Übertragung eines Vektors gemäß einem der Ansprüche 16 bis 19 in individualisierte Zellen oder in Zellgruppen erfolgt, gegebenenfalls in Gegenwart eines weiteren Vektors des Typs Ti-Plasmid, welcher Funktionen aufweist, die diese Übertragung ermöglichen.

## Claims

1. Plant **characterized in that** it carries in its genome one or more genes expressing one or more enzymes of the initial steps of the phytosterol biosynthesis pathway comprising the chain of biochemical reactions whereby mevalonic acid is converted into farnesyl diphosphate, said genes being either not found in the native plant, or originating from said plant, in which case they are present in a number of copies greater than that of the native plant or are modified in their structure or their function compared with those of the native plant.

2. Plant according to claim 1, **characterized in that** said initial step is the conversion of mevalonic acid into mevalonyl 5-phosphate.

3. Plant according to one of claims 1 and 2, **characterized in that** it carries one or more genes coding for one or more mevalonate kinases.

4. Plant according to claim 3, **characterized in that** the mevalonate kinase is encoded by the gene ERG12, or one of its derivatives or fragments expressing a mevalonate kinase activity.

5. Plant according to any one of claims 1 to 3, **characterized in that** it carries one or more genes coding for the mevalonate kinase activity in cruciferous plants, and in particular in Arabidopsis or rapeseed.

6. Plant according to claim 1, **characterized in that** it carries one or more genes coding for a farnesyl diphosphate synthetase.

7. Plant according to claim 6, **characterized in that** the farnesyl diphosphate synthetase is encoded by the yeast gene ERG20, or one of its derivatives or fragments expressing a farnesyl diphosphate synthetase activity.

8. Plant according to claim 1, **characterized in that** it carries one or more genes coding for an AMVP kinase.

9. Plant according to claim 8, **characterized in that** the AMVP kinase is encoded by the gene ERG8, or one of its derivatives or fragments expressing an AMVP kinase activity.

10. Plant according to one of claims 1 to 9, **characterized in that** it belongs to the category of oleoproteinaceous plants.

11. Plant according to one of claims 1 to 10, **characterized in that** it belongs to the cruciferous plant family and in particular is a member of the rapeseed species.

12. Plant according to one of claims 1 to 10, **characterized in that** it is a member of the sunflower species.

13. Plant according to one of claims 1 to 9, **characterized in that** it is a member of the Vigna radiata or egg-plant species.

14. Plant according to one of claims 1 to 10, **characterized in that** it belongs to the Fabaceae family and in particular is a member of the soya species.

15. Plant according to one of claims 1 to 14, **characterized in that** the gene or genes are placed under the control of a promoter inserted close to the gene or genes in such a way as to allow the expression of their biologically active products.

16. Vector for the insertion of at least one gene coding for one or more enzymes of the initial steps of the phytosterol biosynthesis pathway comprising the chain of biochemical reactions whereby mevalonic acid is converted into farnesyl diphosphate in plant cells, **characterized in that** it comprises:
- a gene sequence to be inserted,
- one or more promoter sequences upstream of the gene to be inserted,
- one or more terminator sequences downstream of the gene to be inserted,
- one or more markers allowing the selection of the cells in which the gene is inserted, and
- sequences of a Ti plasmid allowing the transfer of said genes and markers from a bacterium to a plant cell, said genes and markers being oriented and inserted so as to allow their expression.

17. Vector according to claim 16, **characterized in that** the gene codes for a mevalonate kinase.

18. Vector according to claim 17, **characterized in that** the gene is the ERG12 gene.

19. Plasmid pFAB2 according to claim 18, **characterized in that** it has been deposited under the name I-1176 on 14th February 1992 in the Collection Nationale de Culture des Micro-organismes of the Institut Pasteur (CNCM).

20. Method for processing a plant by insertion into its genome of a gene coding for one or more enzymes of the initial steps of the phytosterol biosynthesis pathway comprising the chain of biochemical reactions whereby mevalonic acid is converted into farnesyl diphosphate.

21. Method according to claim 20, **characterized in that** the insertion is performed by transfer into individual cells or groups of cells of a vector according to one of claims 16 to 19, optionally in the presence of another vector of the plasmid Ti type having functions allowing this transfer.
